(19) Europäisches Patentamt
European Patent Office
Office européen des brevets

(11) EP 1 357 181 A1

(12) **EUROPEAN PATENT APPLICATION**
published in accordance with Art. 158(3) EPC

(43) Date of publication:
**29.10.2003 Bulletin 2003/44**

(21) Application number: **01272294.8**

(22) Date of filing: **21.12.2001**

(51) Int Cl.7: **C12N 15/09**

(86) International application number:
**PCT/JP01/11293**

(87) International publication number:
**WO 02/051999 (04.07.2002 Gazette 2002/27)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **21.12.2000 JP 2000388739**

(71) Applicants:
- **Kazusa DNA Research Institute Foundation Kisarazu-shi, Chiba 292-0812 (JP)**
- **Kazusa DNA Research Institute Foundation Kisarazu-shi, Chiba 292-0812 (JP)**

(72) Inventors:
- **OHARA, Osamu, c/o KAZUSA DNA RESEARCH INSTITUTE Kisarazu-shi, Chiba 292-0818 (JP)**
- **NAGASE, Takahiro, KAZUSA DNA RESEARCH INSTITUTE Kisarazu-shi, Chiba 292-0818 (JP)**
- **NEGISHI, Takaaki, MOCHIDA PHARMACEUTICAL CO LTD Tokyo 160-8515 (JP)**
- **MIZUSHIMA, Seiichi, MOCHIDA PHARMACEUTICAL CO LTD Tokyo 160-8515 (JP)**
- **FURUSAKO, Shoji, c/o MOCHIDA PHARMACEUTICAL CO LTD Tokyo 160-8515 (JP)**

(74) Representative: **Wablat, Wolfgang, Dr.Dr. Patentanwalt, Potsdamer Chaussee 48 14129 Berlin (DE)**

(54) **NOVEL ATOPIC DERMATITIS-ASSOCIATED GENE AND PROTEINS**

(57) A gene and/or a protein involved in the prevention or treatment of allergic diseases and dermatitis, in particular atopic dermatitis are provided.

An EST fragment comprising the nucleotide sequence of SEQ ID NO: 1; (a) a DNA comprising the nucleotide sequence represented by SEQ ID NO:2 or (b) a DNA hybridizable with the DNA of SEQ ID NO: 2 under stringent conditions and encoding a protein having a DNA binding activity; and a protein encoded by the DNA are also provided.

Further, the present invention relates to an antisense nucleic acid to the above DNA sequence; a method of searching for an agent capable of controlling the expression of the DNA or the activity of the protein; and a test reagent and a test method for allergic diseases.



Printed by Jouve, 75001 PARIS (FR)

## Description

### TECHNICAL FIELD

The present invention relates to a gene encoding a DNA binding protein, which gene was isolated and identified from a cDNA library from human spleen and involved in the development of atopic dermatitis, and said protein.

### BACKGROUND OF THE INVENTION

Atopic dermatitis is a disease, a main lesion of which is itching eczema with repeated exacerbation and remission. Most patients suffering from atopic dermatitis have atopic diathesis. The atopic diathesis means a diathesis easily developing bronchial asthma, allergic rhinitis and conjunctivitis and atopic dermatitis and producing an IgG antibody. Most important clinical complaint of atopic dermatitis is itching. Often the itching in atopic dermatitis becomes intense suddenly. Then, the skin is torn so as to aggravate exanthem, thereby the itching is further increased and the skin is further torn. Such a vicious circle is repeated so that the skin becomes a chronic eczematous lesion. Therefore, atopic dermatitis should be treated for a long period.

Recently, the number of patients suffering from allergic diseases including atopic dermatitis is increasing in advanced nations. The reason therefore is considered to be nurture. Especially, the number of adult patients suffering from atopic dermatitis is increasing significantly and the establishment of a therapeutic method therefor is requested urgently.

As a therapeutic agent for atopic dermatitis, external steroid agents are though to be most effective. Various steroid agents having different pharmaceutical potency classified into five ranks, i.e. weak, medium, strong, vary strong and strongest, have been used depending on the type and the severity of skin symptom, the site, the age and the like. However, the steroid agent causes a rebound such that it is hardly withdrawn because of a relapse of dermatitis after its application. The therapeutic method for atopic dermatitis becomes complicated due to a significant individual difference in clinical effects and the presence of various folk medicines and the like. Further, patients feel distrust and anxiety for the conventional therapy. Especially they fear using a steroid agent.

A critical agent other than a steroid agent has not been provided, but recently tacrolimus (FK506) is used. Tacrolimus is advantageous since it does not show atrophoderma and telangiectasis which are adverse effects of a steroid agent; it has a poor dependence and rebound after withdrawal as compared with a steroid agent; it is not absorbed from a normal skin; and it can be applied to a circumocular skin with relatively safety.

However, since tacrolimus has a strong immunosuppressive effect, a virus infection on a skin should be cared and tacrolimus cannot be administered to patients under ultraviolet therapy. And, its unsafety including the possibility of a skin cancer during the repeated application for a long period has not been established, thereby its application is limited to refractory patients.

As other agents, antihistamic agents (H1 receptor antagonists) and antiallergic agents are used for reducing skin itching. The latter agents are expected to reduce the amount of applied external steroid agent and lower the rank in pharmaceutical potency thereof, but it takes 2 to 4 weeks from the start of the administration to obtain a desired effect. In case of atopic dermatitis, staphylococci aureus are present on a surface of a diseased skin. Especially many staphylococci aureus are present in an inflammatory lesion such as an erosive skin and the like. For the above reasons, an antimicrobial agent is simultaneously applied.

An atopic dermatitis model is produced in an NC/Jic mouse according to mainly two methods, the first method comprising naturally developing a dermatitis and the second method comprising applying a simple hapten. The NC/Jic mouse is a mouse purified by Kondoh et al. (Experimental Animals, Vol. 6, pp. 107-112 (1957)). It has been known that when NC/Jic mouse is bred without controlling microorganisms in the air, it develops atopic dermatitis-like symptoms. The NC/Jic mouse shows all symptoms characteristic of atopic dermatitis, i.e. hyper-IgE-emia, itching, eczematoid lesions and dry skin. It is reported that a repeated application of a hapten to an NC/Jic mouse causes dermatitis efficiently.

As mentioned above, the number of atopic dermatitis patients is recently increasing and therefore more safe and more effective agents therefor are requested. It is believed that a gene involved in the development of atopy is present in an NC mouse naturally developing a human-like atopic dermatitis (Japanese Society for Immunology, 1999). That is, by identifying an atopic dermatitis-associated gene, a screening method for an effective agent for atopic dermatitis and an antisense of the gene can be provided. And, by analyzing an expression mechanism of the gene to elucidate a diathesis of atopic dermatitis, various diagnostic methods can be newly developed.

An object of the present invention is to clone a gene induced in association with the development of dermatitis, in particular atopic dermatitis, to provide a screening method for therapeutic or prophylactic agent for allergic diseases, a therapeutic agent for allergic diseases using an antisense oligonucleotide, and a gene and a diagnostic probe.

BRIEF DESCRIPTION OF DRAWINGS

Fig. 1 illustrates the analytical results of the EST-AD14 expression status when dermatitis was developed in an NC/Jic mouse and a Balb/C mouse.

Fig. 2 illustrates the results of the development of dermatitis when picryl chloride was applied to an NC/Jic mouse in Example 1.

Fig. 3 illustrates the alignment of the nucleotide sequence of EST-AD14 with that of a human as039 gene in the portion corresponding to EST-AD14.

DISCLOSURE OF THE INVENTION

The present invention provides an EST fragment (EST-AD14) represented by SEQ ID:No. 1 isolated from an NC/Jic mouse which is a mouse developing atopic dermatitis and induced with the development of dermatitis. The expression of the EST fragment is specifically induced upon dermatitis or specifically induced in a skin tissue naturally developing atopic dermatitis. And, the present invention provides a human as039 gene represented by SEQ ID:No. 2 including the nucleotide sequence having a high homology with the EST fragment and isolated from a full length cDNA library from human spleen; and a protein represented by SEQ ID:No. 3 encoded by the cDNA and useful in allergic diseases. Further, the present invention provides a searching method for a compound useful in the treatment of allergic diseases using the EST fragment, the human gene or the protein described above; an antisense nucleic acid; a specific antibody; and a test reagent and a test method for allergic diseases.

<EST-AD14>

EST-AD14 is a fragment capable of isolating a gene specifically induced with dermatitis caused by applying a hapten to an NC/Jic mouse which is known as a mouse developing atopic dermatitis according to Differential Display method (T. Ito et al., FEBS Lett., Vol. 351, p. 231 (1994)). This EST-AD14 is induced with the development of dermatitis in a diseased tissue of an NC/Jic developing dermatitis by applying picric acid or a diseased tissue of an NC/Jic mouse naturally developing dermatitis, while such an expression could not be detected in a normal skin. And, in a Balb/C mouse belonging to a line other than an NC/Jic mouse, the development of dermatitis is also induced (Fig. 1).

Since the expression of EST-AD14 is observed only when dermatitis is developed in a mouse, EST-AD14 is believed to be a fragment corresponding to a part of the gene encoding the dermatitis-associated protein induced with the development of dermatitis, in particular atopic dermatitis. Therefore, the DNA comprising the nucleotide sequence represented by SEQ ID:No. 1 or its partial fragment will be useful as a specific probe for allergic diseases and dermatitis, in particular atopic dermatitis.

<Human gene as039>

The present invention provides gene as039 encoding a protein AD14, which has a DNA binding activity and is induced upon the development of dermatitis, in particular atopic dermatitis, as described below. The human gene as0039 represented by SEQ ID:No. 2 is a gene found in the clone AS0039 having a high homology with EST-AD14 among a cDNA library constructed from human spleen according to the method of Ohara et al. (DNA Research, Vol. 4, p.53 (1997)) and size fractionated. It became clear from the search for homology through the public data base that the human gene as039 is a novel gene. In a clone from human brain obtained in the same way, a novel clone homologous to a part of the human gene as039, KIAA1554 (Gene Bank Accession No. AB046774), is also present.

Although the above gene can be isolated and identified from the cDNA library as described above, the gene may be a DNA obtained by cloning using a genetic engineering technique such as a standard hybridization or a chemical synthetic technique such as a phosphoramidate method based on the sequence as disclosed herein. The form of the gene may be a cDNA, a genomic DNA and a chemically synthesized DNA, however not limited thereto.

If the nucleotide sequence of the as039 is provided, a sequence of an RNA and a sequences of a complementary DNA and RNA are univocally determined. Therefore, it should be understood that the present invention also provides an RNA corresponding to the DNA of the present invention as well as a DNA and an RNA having the sequence complementary to the DNA of the present invention. "DNA" and "polynucleotide" are interchangeably used herein.

The DNA of the present invention also includes a DNA hybridizable with the DNA comprising the nucleotide sequence represented by SEQ ID No. 2 under stringent conditions.

That is, it should be understood that a DNA hybridizable with the DNA sequence represented by SEQ ID No. 2 and encoding a protein having functions substantially equivalent to those of the human protein AD14, especially a protein having a DNA binding activity as a dermatitis-associated protein induced with the development of dermatitis, in particular atopic dermatitis, is included within the scope of the present invention. And, a nucleic acid encoding a protein identical with AD14 and having a sequence different from that represented by SEQ ID No. 2 may occur due to the degeneracy of codon. The nucleic acid is also included within the present invention.

In other words, it should be understood that a DNA sequence partially modified by various artificial treatments such as site-specific mutation; random mutation by treating with a mutagen; mutation, deletion, linkage and the like of the DNA fragment by cleaving with a restriction enzyme are included within the present invention as long as it is hybridizable with the DNA sequence represented by SEQ ID No. 2 and encoding the human protein AD14 or a protein having functions substantially equivalent to those of said protein, especially a protein having a DNA binding activity as a dermatitis-associated protein induced with the development of dermatitis, in particular atopic dermatitis, even if the sequence is different from the DNA sequence represented by SEQ ID No. 2.

The DNA mutant is acceptable as long as it has a homology with the DNA sequence represented by SEQ ID No. 2 of at least 70%, preferably at least 80%, more preferably at least 90%. The homology in DNA sequence can be analyzed by BLAST (J. Mol. Evol. , Vol. 36, pp. 290-300 (1993); J. Mol. Biol., Vol. 215, pp. 403-410 (1990)). The term "hybridizable" means that a DNA is hybridizable with the nucleic acid represented by SEQ ID No. 2 by southern hybridization under stringent conditions. For example, if a probe labeled with DIG Labeling kit (Cat No. 1175033 of Rosche Diagnostics) is used , the hybridization is conducted in a DIG Easy Hyb solution (Cat No. 1603558 of Rosche Diagnostics) at the temperature of, for example, 32°C (preferably 37°C, more preferably 42°C) and the membrane is washed in, for example, 0.5 × SSC solution (containing 0.1% (w/w) SDS) at 50°C (preferably 65°C) (note: 1 × SSC is 0.15 NaCl and 0.015M sodium citrate).

It is believed that the DNA comprising the nucleotide sequence represented by SEQ ID No. 2 or its partial fragment, especially a fragment corresponding to the nucleotide sequence of base Nos. 1 to 419 is useful as a specific probe for allergic disease and dermatitis, in particular atopic dermatitis since a part of the DNA comprising the nucleotide sequence represented by SEQ ID No. 2, especially the nucleotide sequence of base Nos. 1 to 419 has a homology as high as 73.6% with EST-AD14 represented by SEQ ID No. 1 at the nucleotide sequence level. And, the DNA having a length of at least 15 bases and hybridizable with the nucleotide sequence represented by SEQ ID No. 2 or 4 or its complementary sequence under stringent conditions is useful as a test probe or primer for allergic diseases, thereby it is useful as a test reagent. The stringent conditions are as defined above. The suitable stringent conditions can be calculated from a GC content in the sequence according to a manual of DIC Easy Hyb (Cat No. 1603558 of Rosche Diagnostics). For testing allergic diseases, a sample is taken from a subject and the thus-obtained sample per se or an RNA prepared from the sample is subjected to hybridization or PCR using the above probe or primer so as to detect or quantify the expression of the as039 gene in the sample of the subject and compare the expression amount of the subject with that of a healthy. If the expressed amount (i.e. expression level) of the as039 gene of the subject is higher than that of the healthy, said subject is judged to suffer from allergic disease or have an allergic diathesis. A sample to be tested is not limited, examples of which include a body fluid, a tissue, a cell or a cell body of an animal (especially human), and their extract, culture supernatant, smear and slice. The body fluid includes blood, plasma, serum, urine, liquor, lymph, saliva and pleural effusion. As the cell, peripheral blood lymphocyte and the like , can be used.

Allergic diseases mean diseases in which allergic reactions caused by contacting with allergens are involved. Typical examples of allergic diseases includes bronchial asthma, allergic rhinitis, atopic dermatitis, pollenosis and the like. As the allergen, pollen, mite, house dusts, animal's hairs, metals, chemical substances and the like can be exemplified.

The tests for allergic diseases in the present invention include a test for judging whether or not allergic disease-like symptoms are caused by allergic reaction and a test for judging whether or not a subject has an allergic diathesis.

The DNA of the present invention can be used to commercially produce the protein AD14. And, the DNA can be used for testing the presence or absence of the expression of the protein of the present invention in a tissue by labeling with an enzyme or the like. That is, an expression amount of mRNA as an index of an expression amount of the protein of the present invention in a cell is confirmed by using the DNA as a probe so that a cell and cultivating conditions of the cell suitable for the preparation of the protein of the present invention can be determined. In addition, a disease associated with the protein of the present invention can be diagnosed.

Further, an abnormality or polymorphism on the nucleic acid sequence can be tested and/or diagnosed by any method such as PCR-RFLP (Restriction fragment length polymorphism) method, PCR-SSCP (Single strand conformation polymorphism), sequencing method and the like, using a part of the DNA of the present invention as a primer.

And, the DNA of the present invention can be used in gene therapy of a disease in which the expression or the activity of the protein of the present invention is lost, by introducing the DNA of the present invention into an in vivo cell.

The DNA of the present invention is very useful in the preparation of a transformant, the production of a recombinant protein AD14 using said transformant and the search for a compound specifically controlling the expression of the gene as 039.

The transformant of the present invention can be produced according to a method known for those skilled in the art. For example, the DNA of the present invention can be incorporated into a suitable host cell using any one of vectors commercially available or easily obtained by those skilled in the art. Then, the expression of the gene as039 within the host cell can be suitably controlled by placing the gene as039 under the influence of an expression control gene, typical examples of which are a promoter and an enhancer. This technique is suitable for being used in the production of the protein AD14 using the transformed host cell as well as the investigation of mechanisms how to control the expression of the gene as039 and the screening of an agent capable of controlling the expression of the gene.

For example, by contacting any test substance with a cell transformed with the vector containing the gene as 039 under suitable conditions, an agent capable of promoting or inhibiting the expression of the gene as 039 can be searched or evaluated.

By using the DNA of the present invention in combination with a known technique, a transgenic animal can be produced from a suitable animal such as mouse or the like. The transgenic animal is also used in the search or evaluation as well as the transformant. Especially, since the gene as039 or the protein AD14 of the present invention is associated with the development of dermatitis, in particular atopic dermatitis, compounds obtained through the search using the transformant or transgenic animal are expected to be an effective therapeutic or prophylactic agent for allergic diseases and dermatitis, in particular atopic dermatitis.

The EST-AD14 and the gene as039 of the present invention are useful as a diagnostic probe for allergic diseases, various skin diseases, asthma, psoriasis, virus diseases, pancreatitis, bronchitis, nephritis, inflammatory bowel diseases, arteriosclerosis, rheumatoid arthritis and the like. They are also useful in investigation, prevention and therapy of these diseases.

<Protein AD14>

The protein AD14 encoded by the human gene as039 has a deduced amino acid sequence represented by SEQ ID:No. 3. A Zinc finger domain of C3HC4 type essential for a DNA binding activity is present in a portion of amino acid Nos. 222 to 260 and a leucine zipper motif is present in the portion of amino acid Nos. 1087 to 1108. Since the nucleotide sequence corresponding to about 120 amino acid residues corresponding to N-terminal of the protein has a high homology with EST-AD14, it is considered that the human protein AD14 is a protein having a DNA binding activity induced upon the development of dermatitis, in particular atopic dermatitis in human, for example a transcriptional control protein.

Thus, the human protein AD14 has such functions that its expression is induced with the development of dermatitis, in particular atopic dermatitis, that it expresses in an inflammatory lesion and control an inflammation and that it causes a change in gene expression within a cell. Especially, it has an activity of controlling the transcription or the binding to DNA.

The activity of the protein AD14 in binding to DNA can be determined by, for example, sequencing the bound DNA sequence of AD14 according to a method as illustrated in Example 5 and then determining the binding of a probe DNA containing the bound sequence to AD14 according to a method as illustrated in Example 6. And, it is possible to suitably use methods conventionally used by those skilled in the art such as elecrophoretic mobility shift assay (EMSA) (Garner, M. et al., Nucleic Acids Research, Vol. 9, pp. 3047-3060 (1981)) and McKay assay (McKay, R.D. et al., J. Mol. Biol., Vol. 145, pp. 471-488 (1981)). The transcriptional control activity of the protein AD14 can be determined by, for example, detecting a change in expression of the gene in a recombinant capable of inductively expressing AD14 before and after the inductive expression of AD14 according to a microarray method or the like.

A protein having an amino acid sequence wherein substitution, deletion and/or addition of at least one amino acid has occurred in the amino acid sequence represented by SEQ ID:No. 3 is included within the scope of the present invention as long as this protein is functionally equivalent to the above protein AD14, especially it has the activity of binding to DNA as a dermatitis-associated protein induced with the development of dermatitis, in particular atopic dermatitis.

Side chains of amino acid residues which are constitutional elements of a protein are different in terms of hydrophobicity, charge, size and the like, but they are known to have several highly conservative relationships since they do not substantially affect a three-dimensional structure (also called as configuration) of the entire protein. Examples of the substitutions of amino acid residues include glycine (Gly) and proline (Pro); Gly and alanine (Ala) or valine (Val); leucine (Leu) and isoleucine (Ile); glutamic acid (Glu) and glutamine (G1n); aspartic acid (Asp) and asparagine (Asn); cysteine (Cys) and threonine (Thr); Thr and serine (Ser) or Ala; lysine (Lys) and arginine (Arg); and the like. Since Ala, Val, Leu, Ile, Pro, methionine (Met), phenylalanine (Phe), tryptophane (Trp), Gly and Cys are classified as apolar amino acids, they are understood to have similar properties to each other. Non-charged polar amino acids include Ser, Thr, tyrosine (Tyr), Asn and Gln. Acidic amino acids include Asp and Glu. Basic amino acids include Lyr, Arg and histidine (His) . Even if the conservation as defined above is lost, many mutants maintaining functions essential for the protein (in the present invention, the DNA binding activity of the dermatitis-associated protein induced with the development of dermatitis, in particular atopic dermatitis) are known for those skilled in the art. Further, in several similar proteins conserved between different species, it is recognized that they maintain essential functions even if several amino acids are deleted or inserted concentratedly or scatteringly.

Accordingly, a mutant protein resulting from substitution, insertion, deletion or the like of one or more amino acids in the amino acid sequence represented by SEQ ID No. 3 are included within the scope of the present invention as long as it is a protein functionally equivalent to the human protein AD14, especially it is a protein having a DNA binding activity as the dermatitis-associated protein induced with the development of dermatitis, in particular atopic dermatitis.

The term "functionally equivalent" means that a protein in question has at least a binding activity to the sequence to which AD14 binds. The activity is, for example, about 0.1 to 100 times, preferably about 0.5 to 10 times, more preferably 0.5 to 2 times.

The changes in amino acids are found in the nature such as the diversity in sequence between different species or the so-called gene polymorphism. Further, it can be produced artificially according to a known method for those skilled in the art, for example, mutagenesis using a mutagene such as NTG and site-directed mutagenesis using various recombinant gene techniques. The site and the number of the mutation of amino acids are not particularly limited as long as the resultant mutant protein maintains a transcriptional control activity. The mutation number is generally within several tens of amino acids, preferably within 10 amino acids, more preferably within 1 or several amino acids.

The protein of the present invention can be used in searching for an agent capable of binding to said protein and a substance capable of controlling the activity of said protein. The thus-searched agent is expected to be useful as an effective therapeutic or prophylactic agent for allergic diseases and dermatitis, in particular atopic dermatitis associated with the protein of the present invention.

<Antibody>

Further, the present invention provides an antibody capable of binding to the protein AD14 of the present invention. The antibody of the present invention is an antibody specifically recognizing the entire protein AD14 or its partial peptide as an antigen. It includes a monoclonal antibody and/or a polyclonal antibody. And, it may be an antibody belonging to any one of five classes (IgG, IgA, IgM, IgD and IgE) classified by the structure, physical-chemical properties and immunological properties of immunoglobulins or either subclass classified by the type of H chain. Further, it may be a fragment such as $F(ab)'_2$ produced by digesting an immunoglobulin with, for example, pepsin, Fab produced by digesting an immunoglobulin with papain and the like, or a chimera antibody. The antibody is useful in investigation or clinical detection of AD14, clinical therapy of diseases caused by AD14 and the like. And, the antibody can be used for preparing an antibody column useful in the purification of the protein of the present invention and detecting the protein of the present invention in each fraction upon purification.

<Antisense nucleic acid>

The present invention provides the so-called antisense nucleic acid capable of inhibiting the biosynthesis of the protein AD14 at a nucleic acid level *in vivo.* The antisense nucleic acid means a nucleic acid which binds to DNA or RNA involved in carrying a genetic information during either of a transcription stage from a genome region to a pre-mRNA essential for the production of mRNA encoding the protein AD14, a processing stage from the pre-mRNA to a mature mRNA, a stage of passing through a nuclear membrane or a translation stage into a protein so as to affect the normal stream of the transmission of the genetic information and thereby to control the expression of the protein.

It may comprises a sequence complementary to the entire nucleic acid sequence of the gene as039 or either part of the sequence. Preferably, it comprises a nucleic acid (including DNA and RNA) comprising a sequence corresponding to or complementary to the nucleic acid sequence represented by SEQ ID:No. 2 or 4. When mRNA transcripted from the genome region contains an intron structure or a non-translated region at 5 ' or 3'-terminal, an antisense nucleic acid corresponding to or complementary to the sequence of the non-translated region will have functions equivalent to those of the antisense nucleic acid of the present invention.

The antisense nucleic acid of the present invention includes a DNA and an RNA as well as all of derivatives similar to the DNA and the RNA in configuration and functions. The antisence nucleic acid includes a nucleic acid having any other substance bound at 3'- or 5'-terminal, a nucleic acid wherein at least one of bases, sugars and phosphates of the oligonucleotide is substituted or modified, a nucleic acid having a non-naturally occurring base, sugar or phosphate, a nucleic acid having a backbone other than the sugar-phosphate backbone and the like. These nucleic acids are suitable as derivatives in which at least one of a nuclease resistance, a tissue selectivity, a cell permeability and a binding power is improved. That is, the form of the nucleic acid is not limited as long as the nucleic acid can inhibit the activity and the expression of AD14.

And, the antisence nucleic acid having the nucleotide sequence complementary to the nucleotide sequence hybridizable with a loop portion of mRNA forming a stem loop, i.e. the nucleotide sequence of a region forming a stem loop is generally preferable in the present invention. Alternatively, an antisense nucleic acid capable of binding to near a translation initiation codon, a ribosome binding site, a capping site and a splicing site, i.e. an antisense nucleic acid having the sequence complementary to that of these sites is also preferable since generally it can be expected to be very effective in inhibiting the expression.

In order to make the above antisence nucleic acid introduced into a cell and act efficiently, it is preferable that the length of the antisense nucleic acid of the present invention is 15 to 30 bases, preferably 15 to 25 basess, more preferably 18 to 22 bases.

The effect of the antisense nucleic acid of the present invention in controlling the expression can be evaluated by a known method, for example, by determining an inhibitory activity during a translation stage using *in vitro* transcription (Ribo max systems; Promega) together with *in vitro* translation (Rabbit Reticulocyte Lysate Systems; Promega) or an expression amount of a reporter gene such as luciferase and the like using an expression plasmid comprising the reporter gene linked to the DNA containing a 5'-non-translated region, a region near a translational initiation site and a 5'-translation region.

Since the antisense nucleic acid of the present invention can inhibit the expression of the AD14 activity, it is expected that the antisence nucleic acid is useful as an effective therapeutic or prophylactic agent for allergic diseases and dermatitis, in particular atopic dermatitis, in which the protein of the present invention is involved.

## BEST MODE FOR CARRYING OUT OF THE INVENTION

<Nucleic acid>

The DNA of the present invention may be a single strand DNA. Alternatively, it may bind to a DNA or an RNA having the sequence complementary thereto to form a double- or triple-strand. The DNA may be labeled with an enzyme such as horseradish peroxidase (HRPO); a radioactive isotope; a fluorescent substance; a chemiluminescent substance; and the like.

The method for obtaining the DNA of the present invention from a DNA library includes a method comprising screening a suitable genomic DNA library or cDNA library according to a screening method such as a screening method via hybridization and an immunoscreening method using an antibody, amplifying a clone having the desired DNA and cleaving the DNA with a restriction enzyme or the like. In the screening method via hybridization, the hybridization can be conducted for any cDNA library using the DNA having the nucleotide sequence represented by SEQ ID No. 1 or 2 or a part thereof labeled with $^{32}P$ or the like as a probe according to a known method (see, for example, Maniatis, T. et al., Molecular Cloning, a Laboratory Manual, Cold Spring Harbor Laboratory, New York (1982)). The antibody used in the immunoscreening method may be an antibody of the present invention as described below. The novel DNA of the present invention may be obtained by PCR (Polymerase Chain Reaction) using a genomic DNA library or a cDNA library as a template. PCR is conducted for any DNA library according to a known method (see, for example, Michael, A.I. et al., PCR Protocols, a Guide to Methods and Applications, Academic Press (1990)) using a sence or antisence primer prepared based on the nucleotide sequence of SEQ ID No.1 or 2, thereby the DNA of the present invention can be obtained. As the DNA library used in the above methods, a DNA library having the DNA of the present invention is selected and used. Any DNA library can be used as long as it comprises the DNA of the present invention. A commercially available DNA library may be also used. Alternatively, a cDNA library can be constructed according to a known method (see J. Sambrook et al., Molecular Cloning, a Laboratory Manual, 2nd ed., Cold Spring Harbor Laboratory, New York (1989)) by selecting cells suitable for the construction of the cDNA library from cells having the DNA of the present invention.

The DNA of the present invention can be prepared based on the sequence as disclosed herein by a chemical synthetic technique such as a phosphoramidite method.

The recombinant vector having the DNA of the present invention may have any form such as a cyclic form or a linear form. The recombinant vector may have any other nucleotide sequence in addition to the DNA of the present invention, if necessary. The other nucleotide sequence includes an enhancer sequence, a promoter sequence, a ribosome binding sequence, a nucleotide sequence used for amplifying the number of copies, a nucleotide sequence encoding a signal peptide, a nucleotide sequence encoding other polypeptide, a polyA addition sequence, a splicing sequence, a replication origin, a nucleotide sequence of the gene acting as a selective marker and the like. One preferable example of the recombinant vector of the present invention is an expression vector.

In the gene recombination, it is possible to add a translational initiation codon or a translational stop codon to the DNA of the present invention using a suitable synthetic DNA adapter, and to newly produce or delete a suitable restriction site within the nucleotide sequence. This is the technique routinely conducted by those skilled in the art. Such a processing can be suitably and easily conducted based on the DNA of the present invention.

As the vector including the DNA of the present invention, a suitable vector is selected and used depending on the type of a host used. The vector may be a plasmide. Alternatively, various viruses may be used, non-limiting examples of which include bacteriophage, vacuro virus, retro virus, vaccinia virus and the like.

The gene of the present invention can be expressed under the control of a promoter sequence inherent to said gene. Using the expression system, an agent promoting or controlling the transcription of the gene of the present invention can be efficiently searched. Any other suitable expression promoter can be used by linking it to the promoter sequence inherent to said gene upstream of the gene of the present invention or replacing it with the promoter sequence. In this case, the promoter may be selected depending on a host or an object of expression. For example, if a host is E. coli, a T7 promoter, a lac promoter, a trp promoter, a λPL promoter or the like can be used. If a host is a yeast, a PH05 promoter, a GAP promoter, an ADH promoter or the like can be used. If a host is an animal cell, a promoter from SV 40, a retro virus promoter or the like can be used. These lists are not exclusive.

A method for introducing the DNA into a vector is known (see J. Sambrook et al., Molecular Cloning, a Laboratory Manual 2nd ed., Cold Spring Harbor Laboratory, New York (1989)). That is, each of the DNA and the vector is digested with a suitable restriction enzyme and the resultant fragments are ligated with a DNA ligase.

<Protein>

The protein of the present invention can be prepared from various organs naturally expressing said protein. Alternatively, it can be chemically synthesized in a peptide synthesizer (for example, Peptide Synthesizer Model 433A; Applied Biosystems Japan) or it can be produced by recombination method using a suitable host cell selected from prokaryotic cells and eukaryotic cells. However, a genetic engineering technique and a recombinant protein produced thereby are preferable in view of purity.

A host cell to be transformed using the recombinant vector described in the previous section is not limitative. Many cells such as lower cells available in genetic engineering techniques, typical examples of which are E. coli, B. subtilis and S.cerevisiae; and animal cells, typical examples of which are insect cell, COS7 cell, CHO cell and Hela cell, can be used in the present invention.

The transformant of the present invention can be obtained by transforming a suitable host cell using the recombinant vector of the present invention. As the method of introducing the recombinant vector described in the previous section into a host cell, some methods are known, such as an electroporation, a protoplast method, an alkali method, a calcium phosphate precipitation method, a DEAE dextran method, a microinjection method, a method using virus particles and the like (see "Handbook of Genetic Engineering", Special Issue of Experimental Medicines, published by Yodosha Co., Ltd. (March 20, 1991)). Either method may be used.

For preparing the present protein by a genetic engineering technique, the above transformant is cultured to obtain a culture mixture followed by purify the present protein. The transformant can be cultured according to a standard method.

Many textbooks are available, for example, "Experimental Procedures in Microbiology", edited by The Japanese Biochemical Society, published by Tokyo Kagaku Dozin Co., Ltd. (1992)) describing the culture of transformants, for reference.

As a method for purifying the protein of the present invention from the culture mixture, a suitable method is selected among conventional methods for purifying proteins. The conventional methods include salting out, ultrafiltration, isoelectric precipitation, gel filtration, electrophoresis, various affinity chromatographies including ion-exchange chromatography, hydrophobic chromatography and antibody chromatography, chromatofocusing, adsorption chromatography, reverse phase chromatography and the like. If necessary, HPLC systems or the like may be used to conduct several methods in a suitable order.

It is possible to express the protein of the present invention as a fused protein with any other protein or tag such as glutathion S transferase, Protein A, hexahistidine tag, FLAG tag and the like. The thus-expressed fused protein may be separated with a suitable protease such as thrombin, enterokinase and the like. This may be more effective for the preparation of the protein. For purifying the protein of the present invention, conventional methods may be suitably combined. Especially if the protein is expressed in the form of a fused protein, it is preferable to purify according to a method characteristic to such a form. Owing to the use of the activity of the present protein in binding to DNA, the protein can be purified more easily.

One of methods for preparing the present protein by a genetic engineering technique is the synthesis of a cell-free system using a recombinant DNA molecule (J. Sambrook et al., Molecular Cloning 2nd ed. (1989)).

As mentioned above, the protein of the present invention can be prepared in the form of a single protein or a fused protein with any other different protein. The form of the protein of the present invention is not limited to them.

Further, it is possible to transform the protein of the present invention to various forms. For example, it is considered that the present protein can be processed according to various methods known for those skilled in the art such as various chemical modifications to the protein, the binding of the protein to a polymeric substance such as polyethylene glycol and the like, the binding of the protein to an insoluble carrier and the like. And, the presence or absence of glycosylation or the difference in glycosylation degree is found depending on a host cell used. It should be understood that all of the above proteins are included within the scope of the present invention as long as said proteins are a protein functionally equivalent to the protein AD14.

<Transgenic animal>

By using the gene as039 of the present invention, a transgenic non-human mammalian animal can be produced. The transgenic non-human mammalian animal is also included within the scope of the present invention. The transgenic non-human mammalian animal can be produced according to a routine method conventionally used in the production of transgenic animals (see, for example, "Experimental Manual of Genesis, published by Kodansha Scientific Ltd., edited by Motoya KATSUKI under supervision of Tatsuji NOMURA (1987)). That is, the gene or the recombinant vector of the present invention is introduced into a totipotent cell of a non-human animal to produce subjects and thereafter only a subject in which the gene introduced is incorporated in a genome of a somatic cell is selected.

Specifically, in case of a transgenic mouse, a DNA prepared such that the as039 gene can be expressed is directly introduced into a pronucleic oosperm obtained from a normal C57BL mouse. More specifically, a construct is prepared by introducing the as039 gene downstream of a suitable promoter by linking. Thereafter, a linear DNA is obtained by removing the sequence from a prokaryote as much as possible, if necessary. This DNA is directly poured into a pronucleus of the pronucleic oosperm using a fine glass needle.

The oosperm is transplanted in an uterus of another pseudopregnant mouse as an allomother. The pseudopregnant mouse is generally prepared by mating an ICR female mouse with a vasectomized or vasoligated male mouse. A genomic DNA is extracted from a tissue from the transplated embryo and confirmed whether or not the as039 gene is introduced by PCR or southern blotting, thereby a transgenic mouse is obtained.

The so-called "knock-out mouse" can be produced based on the nucleotide sequence of the as039 (or a mouse homologous gene of as 039) . The term "knock-out mouse" used herein means a mouse in which an endogenous gene encoding the protein of the present invention is knocked out (inactivated). The knock-out mouse can be produced by, for example, a positive-negative selection method via homologous recombination (see, for example, US patent Nos. 5,464,764, 5,487,992 and 5,627,059; Proc. Natl. Acad. Sci. USA, Vol. 86, pp. 8932-8935 (1989) ; Nature, Vol. 342, pp. 435-438 (1989)). Such a knock-out mouse is one embodiment of the present invention.

Recently, the production of clone animals by nuclear transplantation in medium or large animals becomes possible.

In this connection, TG and KO animals have been practically produced using this technique. That is, a somatic cell or a germinal cell is subjected to homologous recombination based on the nucleotide sequence of the as039 (or a homologous gene of as039 in each animal) in the same way as that applied to ES cells and then a nucleus is obtained from the resultant cell and used to obtain a cloned animal. This animal is a knock-out animal in which the as039 gene (or a homologous gene of as039 in each animal) is lost. Or, the as039 gene (or a homologous gene of as 039 in each animal)is introduced in any cell of any animal and then the resultant nucleus is used to obtain a clone animal, thereby a TG animal can be produced. Such a knock-out non-human animal and a transgenic non-human animal are one embodiment of the present invention irrespective of its species.

<Antibody>

The antibody of the present invention may be polyclonal or monoclonal. Either antibody can be obtained by referring to a known method (see, for example, "Experimental Procedures in Immunology" , edited by Japan Society for Immunology, published by Japan Society for Immunology), as describe below in brief.

For obtaining the novel antibody, an animal is inoculated with the protein of the present invention and if necessary a suitable adjuvant such as Freund's complete adjuvant (FCA), Freund's incomplete adjuvant (FIA) and the like. If necessary, a booster at an interval of 2 to 4 weeks may be conducted. After the booster, an anti-serum is obtained by taking a blood sample. The protein of the present invention used as an antigen is that obtained in any method as long as it has a purity sufficient to be usable in the preparation of an antibody. A partial polypeptide of the protein of the present invention may be suitably used as an immunizing antigen. If the polypeptide used as an immunizing antigen is a low-molecular weight polypeptide, i.e. a polypeptide comprising about 10 to 20 amino acids, it may be linked to a carrier such as keyhole limpet hemocyanin (KLH) and the like and used as an antigen. Animals to be immunized include those conventionally used in immunological experiments by those skilled in the art such as rat, mouse, rabbit, sheep, horse, chicken, goat, pig, cattle and the like, among which preferably a species capable of producing the desired antibody is selected and used. However it is not limited thereto.

A polyclonal antibody can be obtained by purifying the resultant anti-serum. The purification may be conducted by suitably combining known methods such as salting-out, ion exchange chromatography, affinity chromatography and the like.

A monoclonal antibody is obtained as follows: An anti-body-producing cell such as a spleen cell, a lymphocyte and the like is taken from an immunized animal. The cell is fused with a myeloma cell strain or the like according to a known method using polyethylene glycol, Sendai virus, an eletric plus or the like to produce a hybridoma. Thereafter, a clone producing an antibody which binds to the protein of the present invention is selected and cultured. By purifying a supernatant of the culture of the selected clone, the monoclonal antibody is obtained. The purification may be conducted by suitably combining known methods such as salting-out, ion exchange chromatography, affinity chromatography and the like.

And, the novel antibody is obtained by a genetic engineering technique. For example, an mRNA is obtained from a spleen cell or a lymphocyte of an animal immunized with the protein of the present invention or its partial polypeptide, or from a hybridoma producing a monoclonal antibody against the protein of the present invention or its partial polypeptide. Based on the thus-obtained mRNA, a cDNA library is constructed. A clone producing the antibody which reacts with the antigen is screened and the thus-screened clone is cultured. The desired antibody can be purified from the culture mixture by combined known methods. When the antibody is used for therapy, a humanized antibody is preferable in view of immunogenicity. The humanized antibody can be prepared by immunizing a mouse whose immune system has replaced with a human immune system (see, for example, Nat. Genet., Vol. 15, pp. 146-157 (1997)). Alternatively, the humanized antibody can be engineered using a hypervariable region of the monoclonal antibody (Method in Enzymology, Vol. 203, pp. 99-121 (1999)).

<Antisense nucleic acid>

The antisense nucleic acid and the DNA for a test reagent for allergic diseases can be prepared according to a known method (see, for example, edited by Stanley T. Crooke and Bernald Lableu, in Antisense Research and Applications, published by CRC Publisher of Florida (1993)). If DNA and RNA are native, the antisense nucleic acid and DNA for a test reagent for allergic diseases of the present invention can be obtained by synthesizing in a chemical synthesizer or conducting PCR using the as039 as a template. Alternatively, a part of derivatives such as methyl phosphonate type and phosphorothioate type can be synthesized in a chemical synthesizer (for example, Expedite Model 8909; Applied Biosystems Japan). Then, such a derivative may be synthesized according to a manual attached to the chemical synthesizer and the thus-synthesized product may be purified by HPLC using a reverse phase chromatography or the like, thereby the antisense nucleic acid and the DNA for a test reagent for allergic diseases can be obtained.

When a part of the DNA and the antisense nucleic acid of the present invention is used as a diagnostic or test probe, they are labeled with a radioisotope, an enzyme, a fluorescent substance, a luminescent substance or the like according to a known method. Subsequently, a DNA or an mRNA is prepared from a specimen according to a known method and it is used as a test substance. This test substance is reacted with the labeled probe and then the reaction is washed to remove the labeled probe unreacted. If the test substance contains the gene as039 or RNA, said probe is bound thereto. The presence or absence of the binding formation can be known by using a luminescence, a fluorescent, a radioactivity or the like from the enzyme, a fluorescent substance or a luminescent substance labeled; or a radioisotope as an index.

When the DNA, the antisense nucleic acid or the recombinant vector of the present invention is used in clinical applications, it is preferable to use those having a purity suitable for the use of a medicine according to any pharmaceutically acceptable method.

The DNA, the antisense nucleic acid or the recombinant vector of the present invention may be used by directly dissolving or suspending in a suitable solvent. Alternatively, it may be used after encapsulating in a liposome or incorporating into a suitable vector. If necessary, it may be used in a suitable dosage form such as injections, tablets, capsules, eye drops, creams, suppositories, spray, poultices in which pharmaceutically acceptable adjuvants are added. Examples of the pharmaceutically acceptable adjuvants are a solvent, a base, a stabilizer, a preservative, a solubilizing agent, an excipient, a buffer and the like.

When the DNA, the antisense nucleic acid or the recombinant vector of the present invention is used in the above dosage form, its administration method and its dose can be selected depending on the age and the sex of a patient, the type and severity of the disease. Thus, it may be administered in an amount suitable to improve dermatitis, in particular atopic dermatitis by the suitable method selected from oral, inhalation, transdermal, intravaginal, intraarticular, intrarectal, intravenous, local, intramuscular, subcutaneous and intraperitoneal administrations.

<Screening method>

The present invention relates to a method of screening a substance capable of controlling the activity of the protein of the present invention or the expression of the DNA, which comprises using the protein of the present invention, a transformant expressing said protein, the DNA of the present invention, a recombinant vector comprising said DNA, a transformant produced by transforming with said vector or a transgenic non-human mammalian animal produced by transforming with the DNA of the present invention.
More specifically, the screening method includes:

(1) a method of evaluating DNA binding activity of AD14 in the presence or absence of a test substance, for example a method comprising coexisting the protein of the present invention and a nucleic acid containing a target nucleic acid sequence for said protein and detecting the enhancement or inhibition of the binding of the protein to the nucleic acid by a suitable binding assay to confirm whether the test substance promotes or inhibits the activity of said protein in binding to DNA;
(2) a method comprising coexisting the gene of the present invention and a test substance under a circumstance capable of transcripting said gene and detecting the promotion or inhibition of the transcription of said gene by a suitable method to confirm whether the test substance promotes or inhibits the transcription;
(3) a method comprising using the recombinant vector or the transformant of the present invention and detecting a change in expression of the gene when the protein of the present invention is forcedly expressed according to a microarray method or the like to screen a gene whose expression amount changes with the expression of said protein, i.e. a gene undergoing a transcriptional control by AD14;
(4) a method comprising coexisting a test substance in the above , system (3) and comparing the change in expression of the gene in the presence or absence of a test substance to screen an agent involved in the transcriptional control of the protein of the present invention;
and the like.

An agent capable of controlling the activity of the protein of the present invention means an agonist or antagonist of the protein AD14. The agent capable of controlling the activity of the protein of the present invention may be either an agent enhancing(agonist) or inhibiting (antagonist) the activity of the protein AD14 in binding to DNA. Preferable agent is an antagonist, since it is expected that the antagonist has a therapeutic effect for allergic diseases. An agent capable of controlling the expression of the DNA of the present invention means an agent capable of either promoting or inhibiting the expression of the gene as039. An agent inhibiting the expression is preferable. For confirming whether a test substance controls the activity of the protein of the present invention or the expression of the DNA of the present invention, a difference in the activity of the protein or the expression level of the DNA is determined between the addition and no addition of a test substance in a system capable of confirming the activity of the protein or the expression of the DNA. The expression level of the DNA may be determined on the basis of an expression strength of the gene as 039 into mRNA or the protein. Instead of the expression level of the as039 gene or the protein AD14 per se, an expression level of a reporter gene may be detected. Having function of inhibiting or controlling means that a determined value as to the activity of the protein or the expression level of the DNA in a group with the addition of a test substance is lower than that in a group without the addition of a test substance. For example, the inhibition (control) ratio calculated by the following equation is 10% or higher, preferably 30% or higher, more preferably 50% or higher, even preferably 70% or higher, especially preferably 90% or higher.

inhibition (control) ratio (%) =

[ (determined value of a group without the addition of a test substance) minus (determined value of a group with the addition of a test substance)] / (determined value of a group without the addition of a test substance) * 100

The above determined value is suitably determined depending on the nature of a system capable of confirming the activity of the protein or a system capable of confirming the expression of the DNA. For example, if a system capable of confirming the activity of the protein is that as described in Example 6, a radioactivity may be determined. If a system capable of confirming the expression of the DNA is that as described in Example 9, a fluorescent strength may be determined.

The system as described in Example 9 is a reporter-assay system. In the reporter-assay system, an expression amount of a reporter gene arranged understream of a transcriptional control region is determined to screen an agent affecting the transcriptional control region. Examples of the transcriptional control region include a promoter, an enhancer, a CAAT box, a TATA box and the like generally found in a promoter region. As a reporter gene, a CAT (chloramphenicol acetyl transferase) gene, a luciferase gene, a β-galactosidase gene and the like can be used. The transcriptional control region of the gene as039 can be obtained according to a known method, one of which is illustrated in Example 7.

The gene and the protein of the present invention are dermatitis-associated gene and protein induced with the development of dermatitis, in particular atopic dermatitis. Therefore, compounds obtained by searching using the DNA, the protein, the transformant or the transgenic animal described above are expected to be effective therapeutic or prophylactic agents for allergic diseases and dermatitis, in particular atopic dermatitis.

Non-limiting examples of a test substance include proteins, peptides, oligonucleotides, synthetic compounds, organic low-molecular weight compounds, naturally occurring compounds, fermented products, cell extracts, plant extracts, animal tissue extracts and the like. The test substance may be either new or known.

## EXAMPLES

The present invention will be described in more detail by referring to the following examples which are not to be construed as limiting the scope of the invention.

### Example 1: Cloning of EST-AD14

(1) NC/Jic mouse model developing dermatitis

After an abdomen of an NC/Jic mouse was shaved, 200 μl of an acetone/ethanol (1:3) solution containing 5% picryl chloride was first applied to the mouse and subsequently 200 μl of an olive oil containing 1% picryl chloride was applied thereto twice per week at an interval of two or three days to observe a skin status. In order to evaluate objectively, the skin status was scored based on the following basis.

0.5 --- mild eczema
1.0 --- severe eczema
1.5 --- eczema with erythema
2.0 --- eczema with bleeding

A mild to severe dermatitis was developed after the first application and thereafter the dermatitis was temporarily healed. However, after the third week the dermatitis was again developed (Fig. 2). In mice of the control groups applied with only a solvent, no development of dermatitis was observed. The diseased skin of the mouse applied with picryl chloride and the normal skin of the control mouse were removed on the 60th day and lyophilized at a temperature of -80°C, respectively.

(2) Preparation of RNA from skin specimen

The preparation of an entire RNA from the mouse skin specimen was conducted using TR1zo1 reagent (Invitrogen) according to a manual attached thereto in principle. The lyophilized skin tissue was completely homogenized in 8 ml of TRlzol reagent in an electric homogenizer. After insoluble matter was removed from the homogenate by centrifuging, 1.6 ml of chloroform was added and then vigorously stirred. An aqueous phase was separated by centrifuging. The aqueous phase was collected, to which an equal volume of isopropanol was added and centrifuged to obtain precipitates of an entire RNA. The precipitates were washed with 75% ethanol and then dissolved in a DEPC-treated water to determine a concentration.

(3) DNaseI treatment of entire RNA

DNase treatment was conducted to remove DNA from the entire RNA prepared from the skin tissue. The reaction was conducted using Message Clean kit (Funakoshi Co., Ltd.) according to a manual attached thereto. The reaction was conducted in 57 μl of 1 × DNaseI buffer (included in the kit) containing 40 μg of the entire RNA and 10 units of DNaseI. The reaction was incubated at 37°C for 30 minutes, to which an equal volume of a solution of phenol: chloroform (3:1) was added and vortexed. The mixture was centrifuged at 15, 000 rpm at a room temperature for 5 minutes and an upper layer (aqueous layer) was transferred to a new 1.5 ml tube. A 1/10 volume of 3M sodium acetate (pH 5.2) was added, and then a threefold volume of 100% ethanol and 1 μl of Ethachiminmate (NIPPON GENE) was added and turned invertly. The mixture was centrifuged at 15, 000 rpm at 4°C for 15 minutes to remove a supernatant. The residue was washed with 75% ethanol, dried for 10 minutes and dissolved in a DEPC-treated water to determine a concentration. Until use, the resultant solution was stored at a temperature of -80°C.

(4) Differential display (DD) analysis using entire RNA prepared from skin tissue

The fluorescent differential display (hereinafter abbreviated as "FDD") using the entire RNA prepared from the skin tissue was conducted by referring to the method described in the reference (T. Ito et al., FEBS Lett., Vol. 351, pp. 231-236 (1994)). The entire RNA from the DNaseI-treated skin tissue was reverse transcripted using Superscript Pre-amplification system (Invitrogen) to obtain a cDNA. FDD-PCR was conducted using the cDNA corresponding to 20 ng of the entire RNA per reaction. The reaction liquid had the following composition:

| | |
|---|---|
| cDNA (10 ng of entire RNA equivalent/μl) | 2 μl |
| 10 × AmpliTaqGold buffer | 2 μl |
| 2.5 mM dNTP | 1.6 μl |
| AmpliTaqGold (5U/ml) | 0.1 μl |
| arbitrary primer (30 μM) | 2 μl |
| anchor primer (30 μM) | 2 μl |
| $dH_2O$ | 10.2 μl |
| (total | 20 μl) |

The reaction conditions for PCR were one cycle comprising heating at 94°C for 5 minutes, at 40°C for 5 minutes, at 68°C for 5 minutes; 2 cycle each comprising heating at 94°C for 2 minutes, at 40°C for 5 minutes and at 68°C for 5 minutes; 30 cycles each comprising heating at 94°C for 1 minute, at 60°C for 1 minute and at 68°C for 2 minutes; one cycle comprising heating at 68°C for 27 minutes; and thereafter the temperature was kept at 4°C. The primer pairs used were 50 sets of BamT15A (CCCGGATCCTTTTTTTTTTTTTTTA), BamT15C (CCCGGATCCTTTTTTTTTTTTTTTC) or BamT15G (CCCGGATCCTTTTTTTTTTTTTTTG) as an anchor primer in combination with an arbitrary primer. As the arbitrary primer used, an oligomer comprising 25 nucelotides with Tm value of 60°C was designed and synthesized. The gel electrophoresis was conducted at 15 mA for 150 minutes after a 6% modified polyacrylamide gel was prepared and 3 μl of a sample was applied thereto. The gel plate was stained with SYBR GREEN, scanned by FluorImager SI (Molecular Dynamics) and fluorescently detected to obtain an electrophoregram.

(5) Amplification of bands excised by DD analysis and sequencing

Bands showing the difference between the diseased skin and the normal skin were selected and they were excised from the gel. One (Band 1) of the excised bands was further analyzed. Band 1 was detected in DD analysis using BamT15A (CCCGGATCCTTTTTTTTTTTTTTTA) as an anchor primer and PRMBA-01 (CTGGGACGACATGGAGAAGATCTGG) as an arbitrary primer. The expression of Band 1 was observed in the diseased skins of two subjects, but such an expression was not observed in the normal skin. In order to sequence Band 1, the gel containing Band 1 was excised, stored in a TE solution and heated at 99°C for 10 minutes so that the gel was eluted. PCR of 30 cycles each comprising heating at 94°C for 30 seconds, at 55°C for 30 seconds and at 72°C for 1 minute was conducted using the TE solution as a template and the primer identical with that used in the FDD method so as to amplify the DNA fragment of 613 bp. The thus-amplified DNA fragment was cloned using a plasmid vector pBluescript II SK+ (Strategene) and the nucleotide sequence of the DNA fragment (EST-AD14) was sequenced according to a standard method. The sequence was shown in SEQ ID:No. 1. It was found that this fragment is a part of the protein translation region and there was neither N-terminal nor C-terminal within this fragment.

(6) Homology analysis of EST-AD14

Homology was studied by searching the agreement in a local sequences using BLAST (Altschul SF., J. Mol. Evol., Vol. 36, pp. 290-300 (1993); Altschul SF., J. Mol. Biol., Vol. 215, pp. 403-410 (1990)). The homology study of EST-AD14 sequence to Genbank gene database (http://www.ncbi.nlm.nih.gov/) was conducted using the blastn program comparing said EST-AD14 sequence with forward and inverted repeats of a sequence in question. As the result, a gene sequence significantly homologous with EST-AD14 was not found. Consequently, EST-AD14 was believed to be a novel sequence.

(7) Analysis of expression specificity of EST-AD14

The expression specificity of EST-AD14 in an NC/Jic mouse skin was confirmed. Using a skin specimen of an NC/Jic mice different from that used in FDD method, an entire RNA was prepared. RT-PCR was conducted using the entire RNA as a template and a specific primer prepared from the EST-AD25 gene sequence. As the result, the expression of EST-AD25 was induced with the development of the dermatitis in the skin tissue of the NC/Jic mouse developing dermatitis by applying picryl chloride (hapten) (Fig. 1; lanes 7, 8, 11, 12 and 13) and the skin tissue of the NC/Jic mouse naturally developing dermatitis (Fig. 1; lane 6), but such an expression could not be detected in the normal skin tissue (Fig. 1; lanes 1, 2, 5 and 14), as shown in Fig. 1. This supported the results of the FDD method. And, the expression of EST-AD14 was also found in a skin tissue of a Balb/C mouse applied with picryl chloride (Fig. 1; lanes 3 and 4).

<u>Example 2: Cloning of human gene as 039</u>

(1) Construction of cDNA library of human spleen cell

The construction of a cDNA library from human spleen was conducted according to the method of Ohara et al. (DNA Research, Vol. 4, pp. 53-59 (1997)). Specifically, a double-stranded cDNA was synthesized in SuperscriptII reverse transcription enzyme kit (Invitrogen) using an oligonucleotide having NotI site (GACTAGTTCTAGATCGCGAGCGGCCGCCC(T15); Invitrogen) as a primer and a human spleen mRNA (Clontech) as a template. The cDNA was ligated to an adapter having SalI site (Gibco BRL). Thereafter a DNA fragment having 3kb or more was purified by digesting with Not I and subjecting to electrophoresis on 1% low-melting point agarose.

The thus-purified cDNA fragment was ligated to a pBluescript II SK+ plasmid treated with a SalI-NotI restriction enzyme. The recombinant plastmid was introduced in E. coli (Electromax DH10B strain; Invitrogen) by electroporation. Then, about 2,000 recombinants were selected from the thus-constructed cDNA library and both terminal DNA sequences of these clones were determined. All nucleotide sequences of the cDNA of about 100 clones containing the new gene were determined.

For sequencing, a DNA sequencer (ABI PRISM377; Applied Biosystem) and a reaction kit of the same company were used. Shotgun clone was sequenced according to a dye terminator method to determine the most of the sequence. Some parts of the nucleotide sequence were determined by primer walking method after synthesizing an oligonucleotides based on the nucleotide sequences as determined. The thus-obtained full length cDNA sequence was registered in data base. This was used as the data base of the full length cDNA sequence from human spleen.

(2) Analysis of homology with human full length cDNA library data base

In order to obtain a cDNA containing a sequence homologous with EST-AD14, the homology search of the data base of the full length cDNA sequence from human spleen was carried out. The search was conducted using a blastx program translating reading frames in forward and reverse direction of the EST-AD14 nucleotide sequence so as to search a homology. As the result, Nos. 138 to 568 bases of EST-AD14 and Nos. 1 to 419 bases of the human cDNA clone No. AS00039 (plasmid pAS39) showed a homology as high as 73.6% (Fig. 3). The clone AS00039 contained a cDNAcomprising the nucleotide sequence of 4616 bases represented by SEQ ID NO: 4, containing an open reading frame comprising the nucleotide sequence of 4299 bases represented by SEQ ID NO:2 encoding a novel protein which comprises 1432 amino acids represented by SEQ ID NO:3. A motif of the amino acid sequence of the human AD14, the novel protein comprising 1432 amino acids represented by SEQ ID NO:3, was analyzed, and a zinc finger domain of C3HC4 type was found in a portion of amino acid residues Nos. 222 to 260. And, a leucine zipper domain was found in a portion of amino acid residues Nos. 1087 to 1108. From these facts, the human AD14 protein was believed to be a novel protein having a DNA binding activity. The plasmid pAS39 was deposited in International Patent Organism Depositary (IPDO) of National Institute of Advanced Industrial Science and Technology as FERM P-18140 on December 14, 2000 and thereafter it was transferred to the international deposition as FERM BP-7824 on December 12, 2001.

<u>Example 3 : Analysis of expression specificity of human gene as039</u>

The tissue expression specificity of the human gene as039 was analyzed. PCR was conducted using the cDNA library of each human organ as a template and specific primers (sense primer: 5'-AGTTCAAGGTACAAAGCGGA-3', antisense primer: 5'-AA.AGGCACCAGTCCATGATC-3' ) prepared based on the nucleotide sequence of the gene as039. As the result, the significant expression was found in spleen, lung, liver, kidney, spleen and placenta. A weak expression was found in thymus, peripheral blood lymphocyte, heart, small intestine and ovary. And, no expression was found in brain, large intestine, prostate, skeletal muscle and testis (Table 1).

Table 1

| expression specificity of human gene as039 in different organs | |
|---|---|
| thymus | + |
| spleen | ++ |
| peripheral blood lymphocyte | + |
| brain | - |
| heart | + |
| lung | ++ |
| liver | ++ |
| kidney | ++ |
| spleen | ++ |
| large intestine | - |
| small intestine | + |
| skeletal muscle | - |
| prostate | - |
| testis | - |
| ovary | + |
| placenta | ++ |

Example 4 : Expression in mammalian cell

(1) Construction of expression plasmid pHAS39

A plasmid for expressing the human AD14 protein in a mammalian cell was prepared according to the following method.

The pAS39 plasmid prepared by constructing the human full length cDNA library was digested with restriction enzymes ApaI and NotI. The resultant DNA fragment of about 4.6 kbp was end blunted using a DNA blunting kit (Takara Shuzo Co., Ltd.). A pcDN44/HisMAX A (Invitrogen) was digested with a restriction enzyme EcoRV, dephosphated with alkaline phosphatase and subjected to electrophoresis on agarose gel to collect a DNA fragment of about 5.3 kbp as a vector. To the vector fragment was routinely ligated the previously collected DNA fragment of about 4.6 kbp. A JM109 competent cell (Takara Shuzo Co., Ltd.) was transformed to obtain the desired plasmid (pHAS39).

(2) Confirmation of expression of human AD14 protein using COS-1 cells

The expression plasmid pHAS39 was introduced into COS-1 cells according to the following method to express the protein.COS-1 cells were inoculated in a 6-well plate at the concentration of $3.0 \times 10^5$ cells/well and cultured under the condition of 5% $CO_2$ and 37°C for 24 hours. Next day, 6 µl/well of FuGENE6 (Rosche Diagnostics) and 1 µg/well of PHAS39 were mixed according to the provider's protocol, and the mixture was added to the COS-1 cells. The cells were cultured under the condition of 5% $CO_2$ and 37°C for 72 hours and then the supernatant was removed. The cells were washed with PBS- and dissolved in 100 µl/well of ElectroPure Reagent Tris-SDS Sample Buffer (2x concentration) (Daiichi Pure Chemical Co., Ltd.). Then, 10 µl of the resultant solution was subjected to electrophoresis on 5 to 20% gradient SDS polyacrylamide gel and the protein was transferred onto Pall FluoroTrans W Membrane (Pall Corporation) to conduct the western blotting. A Penta.His Antiboty (QIAGEN) was used as a primary antibody and an HEP-labeled rabbit anti-mouse IgG (DAKO) was used as a secondary antibody. The chemical luminescence was detected using ECL detection reagent (Amersham Biotec). As the result, the expression of the protein of about 150 kDa could be confirmed.

(3) Purification of human AD14 protein using COS-1 cells

The expression of the protein was conducted by introducing the expression plasmid PHAS39 into COS-1 cells according to the following method.

50 mL of FuCENE6 (Rosche Diagnostics) was mixed with 12.5 mg of the above plasmid DNA according to the provider's protocol and the mixture was added to COS-1 cells in a growth semi-confluent condition in a 150 $cm^2$ flask. The cells were recovered after cultured under the condition of 5% $CO_2$ and 37°C for 72 hours. The thus-recovered cells were suspended in PBS-, homogenized in an electric homogenizer and centrifuged to separate a soluble fraction as a cytoplasmic fraction. This fraction was applied to a histidine tag affinity column chromatography in a commercially available nickel column ProBond. The equilibration was well conducted with 20 mM phosphate buffer + 500 mM NaCl, pH 7.8. Thereafter, the culture supernatant which had been dialyzed was applied to the nickel column. Most of the cytoplasmic protein was passed through the column. It was washed well and subjected to the gradient elution using 20 mM phosphate buffer + 500 mM NaCl, pH 4 . 0 . Thereafter, the eluted fraction was reacted with a commercially available enterokinase (Enterokinase Cleavage Capture kit; Novagen) at 20°C for 24 hours to obtain a human AS14 protein which was considered to be of active type. This sample was applied to the above affinity column. The fraction passed through the column was dialyzed against PBS- and concentrated to obtain an active-type PAS39 purified specimen. By silver staining, this specimen showed a band at the position of the molecular weight of about 150 kDa.

Example 5 : Searching of DNA sequence bound to a human AD14

A Zinc finger domain of C3HC4 type was found in a portion of amino acid residue Nos. 222 to 260 of the amino acid sequence of the human AD14 protein. And, a leucine zipper domain is found in a portion of amino acid residue Nos. 1087 to 1108 of the amino acid sequence. These facts suggest that the human AD14 may bind to DNA and function as a transcription factor. If a bound sequence recognized by the human AD14 can be determined, a group of genes controlled by the human AD14 can be elucidated.

A DNA sequence bound to the human AD14 can be elucidated by PCR-based binding site screening (Koering et al. , Nucleic Acid Research, Vol. 28, pp. 2519-2526 (2000)). Specifically, an oligonucleotide comprising 40 bases having the sequence GTACGTATCTAGATC($N_{10}$)GCAAGCTTCTA (wherein N is a mixed base of G, C, A and T) is mixed with the human AD14 protein labeled with biotin so that the oligonucleotide having the sequence bound to human AD14 forms a complex with the human AD14 protein. An oligonucleotide - AD14 complex is recovered using a carrier to which streptoavidine is linked. And, an oligonucleotide as simultaneously recovered is amplified by PCR. By repeating this process, the oligonucleotide having the sequence bound to the human AD14 is concentrated and sequenced.

Example 6 : Screening of inhibitor for AD14 - DNA binding

An inhibitor for AD14 - DNA binding can be screened according to the following method. Specifically, the recombinant AD14 protein is expressed by introducing the pHAS39 plasmid into COS-1 cells and purified through a nickel column. Thereafter, the thus-purified AD14 protein is labeled with biotin using a biotin protein labeling kit (Rosche Diagnostics). A DNA having a length of about 10 to several tens base pairs containing the sequence bound to AD14 is provided, and it is labeled with $^{31}P$ to make a probe DNA. The AD14 labeled with biotin, the probe DNA and an inhibitor to be tested are incubated in a 96-well plate coated with streptoavidin. After the plate is washed with a surfactant containing buffer, a radioactivity remaining on the plate is determined. A substance showing the lowering in remaining radioactivity is screened as an inhibitor for the binding.

Example 7 : Cloning of AD14 promoter region

Cloning of an AD14 promoter region is conducted by screening a human genome library using a 5'-terminal DNA fragment of the cDNA as a probe. Specifically, pHA39 is digested with a restriction enzyme ApaI-SacI and subjected to electrophoresis on agarose gel to recover a DNA fragment of about 0.4 kbp. The thus-recovered DNA fragment is labeled with $^{32}P$ using a random primer labeling kit (Takara Shuzo Co., Ltd.) to make a probe DNA. A human genome library (Clontech) is subjected to a plaque formation on a soft agar according to a routine method, and phage particles are transferred and immobilizd onto a nylon membrane. The nylon membrane on which the phages are immobilized and the probe DNA are incubated in a rapid hybribuffer (Amersham Biotech) at 65°C and washed to determine the remaining radioactivity using BAS2500 (Fuji Photo Film Co., Ltd.). Positive plaques are isolated and their nucleotide sequence was determined so that a DNA fragment having a sequence from the upstream of the translation initiation site is cloned as an AD14 promoter region.

Example 8 : Construction of plasmid PHAS39-G for reporter gene assay

The DNA fragment of the AD14 promoter region obtained in the above is amplified by PCR and ligated to a pGlow-TOPO vector (Funakoshi Co., Ltd.) according to a routine method. A JM109 competent cell (Takara Shuzo Co. Ltd.) is transformed to obtain the desired plasmid pHAS39-G.

Example 9 : Screening of agent controlling the transcription of AD14

The plasmid pHAS39-G prepared above is transfected into COS-1 cells using FuGENE6 (Rosche Diagnostics) and the cells are incubated in the presence of a test substance on a 96-well plate. After the incubation, a fluorescence strength of each well is determined. A substance showing the change in fluorescence strength as compared with a control group, in which any substance is not added, is screened as an agent capable of controlling the transcription of AD14.

EFFECT OF THE INVENTION

The present gene is a dermatitis-associated gene induced with the development of a human dermatitis, in particular atopic dermatitis. An agent capable of controlling the expression of the present gene can be used as a therapeutic agent for allergic diseases and dermatitis, in particular atopic dermatitis. Therefore, the present invention can be used in screening for such a therapeutic agent. And, an antisense nucleic acid of the present gene or a vector expressing the nucleic acid can be also used as a therapeutic agent for allergic diseases and dermatitis, in particular atopic dermatitis. Further, the present invention can be used as a diagnostic probe. Therefore, the present gene is useful for studying, preventing and treating diseases such as allergic diseases, various skin diseases, asthma, psoriasis, virus diseases, pancreatitis, bronchitis, nephritis, inflammatory bowel diseases, arteriosclerosis, rheumatoid arthritis and the like.

## Claims

1. A DNA comprising the nucleotide sequence represented by SEQ ID NO:1.

2. The following DNA (a) or (b):

   (a) a DNA comprising the nucleotide sequence represented by SEQ ID NO:2; or
   (b) a DNA hybridizable with the DNA of SEQ ID NO:2 under stringent conditions and encoding a protein having a DNA binding activity.

3. A protein encoded by the DNA as defined in claim 2.

4. The following protein (a) or (b):

   (a) a protein comprising the amino acid sequence represented by SEQ ID NO:3; or
   (b) a protein having an amino acid sequence wherein deletion, substitution or addition of one or more amino acids has occurred in the amino acid sequence of SEQ ID NO:3 and having a DNA binding activity.

5. An antisense nucleic acid inhibiting the expression of the protein as defined in claim 3 or 4.

6. An antisense nucleic acid as defined in claim 5 wherein the nucleic acid sequence is a complementary sequence to the entire or a part of the DNA as defined in claim 1 or 2.

7. A recombinant vector including the DNA as defined in claim 1 or 2.

8. A transformant produced by transformation with the recombinant vector as defined in claim 7.

9. An antibody against the protein as defined in claim 3 or 4 or its partial peptide.

10. A method of screening for an agent capable of controlling an activity of the protein as defined in claim 3 or 4 among test substances, which comprises contacting the protein or the transformant as defined in claim 8 expressing the protein with the test substances and then detecting a change in activity of the protein in binding to DNA.

11. A method of screening for an agent capable of controlling the expression of the DNA as defined in claim 2 among test substances, which comprises contacting the recombinant vector as defined in claim 7 or the transformant as defined in claim 8 with the test substances and then detecting a change in expression level of the DNA as defined in claim 2.

12. A method of screening for a gene whose expression amount varies depending on the expression of the protein as defined in claim 3 or 4, which comprises expressing the protein in the transformant as defined in claim 8.

13. A method of screening for an agent capable of controlling an activity of the protein as defined in claim 3 or 4 among test substances, which comprises contacting the transformant as defined in claim 8 expressing the protein with the test substances and then detecting a change in expression level of a gene whose expression amount varies depending on the expression of the protein.

14. A test reagent for allergic diseases comprising a DNA having a chain length of at least 15 bases hybridizable with the nucleotide sequence represented by either SEQ ID NO :2 or 4 or its comlementary sequence under stringent conditions.

15. A test method for allergic diseases comprising detecting an expression level of the DNA as defined in claim 2 in a sample taken from a subject using the reagent as defined in claim 14.

Fig. 1

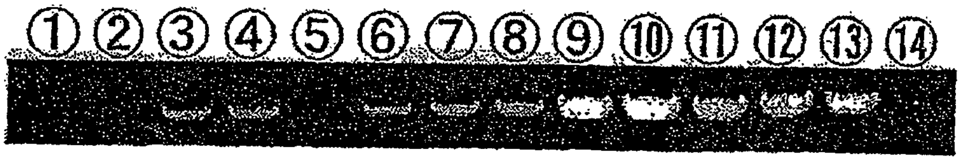


① Balb-c/hapten-, skin
② 〃
③ Balb-c/hapten+, skin
④ 〃
⑤ NC/Jic/hapten-, skin
⑥ 〃 (naturally developing mild dermatitis)
⑦ NC/Jic/hapten+, skin
⑧ 〃
⑨ NC/Jic/hapten-, adrenal
⑩ NC/Jic/hapten+, adrenal
(①–⑩: conventional)

⑪ NC/Jic/hapten+, skin
⑫ 〃
⑬ 〃
⑭ NC/Jic/hapten-, skin
(⑪–⑭: SPF)

Fig. 2

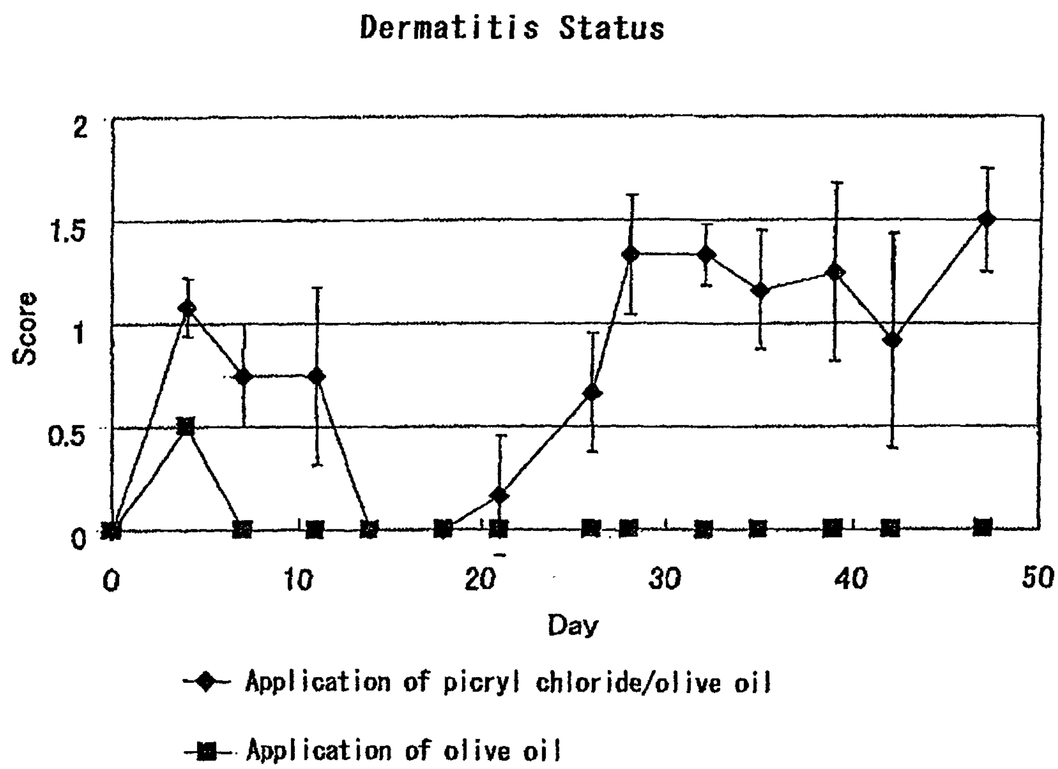
Dermatitis Status
2
1.5
1
0.5
0
Score
0
10
20
30
40
50
Day
Application of picryl chloride/olive oil
Application of olive oil

Fig. 3

```
EST-AD14  GGACTTCCTGCTCTTGACCATGAAAGTGTCTTCAAGGGAGGAATTAATGTTTCTGCAGAT
as039     GGATTTCATTCTCTTGACCATGCGTGTGTCAACGGAGGAGGAATTAAAGTTTCTGCAGAT
          *** *** * ************   *****  *   *********** ************

EST-AD14  GGCCCTGTGGTCCTGCCTCCGTGAGCTCCAAGAGGCTTCAGGAACACCTGATGAAACTTA
as039     GGCTCTGTGGTCCTGCACTAGGAAACTGAAAGCGGCGTCAGAAGCGCCCGAGGAA-----
          *** ************    *  * **  *** *** **** * * ** ** ***

EST-AD14  TAAGTTTCCTCTCTCCTTGCCCTGGGTGCACCTTGCCTTTCAGCACTTCAGGACCCGGCT
as039     GAGGTTT-------CCTTACCGTGGGTGCACCTTGCCTACCAGCGTTTCAGAAGCCGTCT
           * ****       **** ** ****************  ****  ***** * *** **

EST-AD14  ACAGAACTTCTCCAGAATTTTGACCATTCACCCCCAGGTTCTGAGCAGCCTCAGTCAAGC
as039     GCAGAACTTTTCCAGAATCCTGACCATCTACCCTCAGGTTCTCCACAGCCTGATGGAAGC
           ******** ********  *******  **** ********   ****** *   ****

EST-AD14  AGCAGAGAAGCAC-AGCTTGGCTGGGTGTGAAATGACACTGGATGCATTTGCAGCAATGG
as039     CCGTTGGAACCATGAGCT-GGCTGGATGTGAGATGACCCTGGACGCATTTGCCGCAATGG
                *** **  **** ****** ***** ***** ***** ******** *******

EST-AD14  CCTGCGCTGAAATGCTCAAGGGAGACCTCCTGAAGCCAAGCCCCAAGGCTTGGTTACAGC
as039     CCTGCACGGAGATGCTGACAAGAAACACCCTGAAGCCCAGTCCCCAGGCGTGGCTACAGT
          ***** * ** ***** *   ** **  ********* ** *** **** *** *****

EST-AD14  TTGTGAAGAATCTGTCCACGCCACTGGAGCTCGTCTGCTCCGAAGGGTACTTGTGTGACA
as039     TGGTGAAGAATCTTTCCATGCCGCTGGAGCTCATCTGCTCCGATGAGCACATGCAAGGCA
          * *********** **** *** ********* ********** * * ** **   * **

EST-AD14  GTGGGAGCATGA
as039     GCGGGAGCCTGG
          * ****** **
```

**INTERNATIONAL SEARCH REPORT**

International application No.
PCT/JP01/11293

A. CLASSIFICATION OF SUBJECT MATTER
(See extra sheet.)

According to International Patent Classification (IPC) or to both national classification and IPC

B. FIELDS SEARCHED

Minimum documentation searched (classification system followed by classification symbols)
Int.Cl$^7$ C12N15/00-15/90

Documentation searched other than minimum documentation to the extent that such documents are included in the fields searched

Electronic data base consulted during the international search (name of data base and, where practicable, search terms used)
GenBank/EMBL/DDBJ/GeneSeq
WPI(DIALOG)
BIOSIS(DIALOG)

C. DOCUMENTS CONSIDERED TO BE RELEVANT

| Category* | Citation of document, with indication, where appropriate, of the relevant passages | Relevant to claim No. |
|---|---|---|
| A | OHMEN, J.D. et al., Overexpression of IL-10 in atopic dermatitis. Contrasting cytokine patterns with delayed-type hypersensitivity reactions., J.Immunol., 1995, Vol.154, No.4, p.1956-63 | 1-15 |
| A | TANG, M.L.K. et al., Spontaneous expression of IL-4 mRNA in lymphocytes from children with atopic dermatitis., Clin.Exp.Immunol., 1994, Vol.97, No.3, p.491-8 | 1-15 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 March, 2002 (27.03.02) | 09 April, 2002 (09.04.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP01/11293

Continuation of A. CLASSIFICATION OF SUBJECT MATTER
(International Patent Classification (IPC))

Int.Cl[7] C12N15/09, C07K14/47, C07K16/18, C12N1/15, C12N1/19, C12N1/21, C12N5/10, C21P21/02, C12Q1/02, C12Q1/68, A61K31/713, A61K48/00, A61P1/04, A61P1/18, A61P9/10, A61P11/00, A61P11/06, A61P13/12, A61P17/00, A61P17/06, A61P29/00, A61P31/12, G01N33/15, G01N33/50, G01N33/53//(C12P21/02, C12R1:91)
(According to International Patent Classification (IPC) or to both national classification and IPC)

SEQUENCE LISTING

<110> Mochida Pharmaceutical Co., Ltd.

<110> Kazusa DNA Research Institute

<120> Novel gene and protein related to allergic dermatitis

<130> SAP-681-PCT

<150> JP 2000388739

<151> 2000-12-21

<160> 4

<170> PatentIn Ver. 2.1

<210> 1

<211> 568

<212> DNA

<213> Mus musculus

<400> 1

gtgcaggctc agtatatcac agacactgag ggattatcga agaagttcgt ggaaattttc 60
cagaaaactc ccttgggcgt gtttctcgct cagttccctg tggcacagca gcaaaaactc 120
ctgcagagct acttgaagga cttcctgctc ttgaccatga aagtgtcttc aagggaggaa 180
ttaatgtttc tgcagatggc cctgtggtcc tgcctccgtg agctccaaga ggcttcagga 240
acacctgatg aaacttataa gtttcctctc tccttgccct gggtgcacct tgcctttcag 300
cacttcagga cccggctaca gaacttctcc agaattttga ccattcaccc ccaggttctg 360
agcagcctca gtcaagcagc agagaagcac agcttggctg ggtgtgaaat gacactggat 420
gcatttgcag caatggcctg cgctgaaatg ctcaagggag acctcctgaa gccaagcccc 480
aaggcttggt tacagcttgt gaagaatctg tccacgccac tggagctcgt ctgctccgaa 540
gggtacttgt gtgacagtgg gagcatga 568

<210> 2

<211> 4299

<212> DNA

<213> Homo sapiens

<400> 2

atgcgtgtgt caacggagga ggaattaaag tttctgcaga tggctctgtg gtcctgcact 60
aggaaactga aagcggcgtc agaagcgccc gaggaagagg tttccttacc gtgggtgcac 120
cttgcctacc agcgtttcag aagccgtctg cagaactttt ccagaatcct gaccatctac 180
cctcaggttc tccacagcct gatggaagcc cgttggaacc atgagctggc tggatgtgag 240
atgaccctgg acgcatttgc cgcaatggcc tgcacggaga tgctgacaag aaacaccctg 300
aagcccagtc cccaggcgtg gctacagttg gtgaagaatc tttccatgcc gctggagctc 360
atctgctccg atgagcacat gcaaggcagc gggagcctgg cccaggctgt catcagggaa 420
gtcagagccc agtggagtcg gattttctcc accgcactct tcgtggagca cgtgctccta 480
ggaaccgaga gccgcgtccc cgagttacag gggctggtga ccgagcacgt cttcttacta 540
gacaagtgtc ttcgagagaa ctctgacgtg aagacgcacg ggccttttga ggccgtgatg 600
cgcactctct gtgaatgcaa ggagacagcc agcaagaccc tcagcaggtt tgggattcag 660
ccgtgctcca tctgcctggg agatgcaaag gaccccgtct gtctgccctg cgaccacgtg 720
cactgcctgc gctgcctcag ggcctggttt gcctcagagc agatgatatg cccctactgt 780
ttaactgcct tgccagacga attctctcca gctgtttccc aagcgcacag ggaagccatt 840
gaaaagcatg cccgcttccg gcagatgtgc aacagtttct tcgtagacct ggtgtccacc 900
atttgcttca aggacaacgc tccgcctgag aaggaagtga ttgagagcct gctctctctc 960
ctcttcgtcc aaaaggggcg cttaagagat gctgcccaga gacactgtga acacacaaaa 1020
tctctctctc cattcaatga tgttgtggat aagactcctg tcatccgctc agtgatactg 1080
aaactgcttt tgaagtacag ctttcatgat gtaaaagatt atattcagga atatttgacc 1140
ctgttaaaaa agaaagcatt cataactgaa gataaaactg aactgtacat gctcttcatc 1200
aactgcctgg aggattcaat acttgagaag accagtgctt actccagaaa tgatgaactg 1260
aaccacctag aagaggaagg tcgtttcctt aaggcatatt ctccagcaag ccggggccga 1320
gagcctgcca acgaggcctc ggttgaatac ctgcaagagg tggcccggat ccgcctctgc 1380
ctcgacagag ctgcagattt cctctcggag cctgagggag gcccagagat ggccaaggag 1440
aagcagtgct acctgcagca agtcaagcag ttctgtatcc gggtggagaa cgactggcac 1500
cgggtgtacc tggtgcggaa gctcagcagc cagcggggga tggagttcgt gcagggcctc 1560

tccaagcccg gccgcccgca ccagtgggtg tttcccaagg acgttgtcaa gcagcagggg 1620
ctgcggcagg accacccagg ccagatggat aggtacctgg tgtacggcga tgaatacaag 1680
gctctccgtg atgctgtggc caaagctgtc ctcgagtgca agccactggg cattaagact 1740
gctctgaagg cctgcaagac cccccaaagc cagcagtcag cctacttcct gttaacactg 1800
tttagagagg tggctatttt gtacagatcc cacaatgcaa gcctccaccc cacgccagag 1860
caatgtgaag ctgtgagcaa attcattggc gaatgcaaga tcctttcacc tcctgatatc 1920
agccgttttg caacatcgct cgtggacaat tctgtgccat tgttgagggc ggggcctagt 1980
gacagcaacc ttgatggaac ggtgacagaa atggccattc atgctgcagc cgtccttctg 2040
tgtggacaga atgaactctt ggagccccta aagaatctgg ccttctcccc agccaccatg 2100
gcgcatgctt ttcttccaac catgcctgaa gacttgctgg ctcaagctcg gaggtggaag 2160
ggtctggagc gagtccactg gtacacttgt cccaacggcc atccttgctc cgtgggagag 2220
tgtggcaggc cgatggaaca gagcatctgc attgactgcc atgcgccgat tggaggcatt 2280
gaccacaaac ctcgggacgg ctttcatctg gtcaaagaca aggcagacag aacgcagacc 2340
ggccacgtgc tgggcaaccc gcagcggaga gacgtggtga catgtgaccg agggctgccc 2400
ccagtggtct tcctccttat ccggctactc actcacttgg ctctgcttct gggagcgtcc 2460
cagagttccc aggctctgat aaacatcatt aagcctccag tgagggatcc aaaaggcttt 2520
ctgcagcagc acatcctgaa ggacctggag cagttggcca agatgctggg acacagtgcc 2580
gacgagacca tcggcgtggt ccacctcgtc ctgcgcaggc ttctccaaga gcagcaccag 2640
ctctctagca gaaggctttt aaattttgac acagaattgt caactaaaga aatgaggaac 2700
aactgggaaa aggaaatcgc agctgtgatt tctcctgaac tggagcatct agataaaacc 2760
cttcccacca tgaataatct catcagccaa gataagcgta tcagctctaa ccctgtggcc 2820
aaaataatat atggtgaccc agtgaccttc ctgccccacc tgccccggaa aagtgtggtc 2880
cattgctcta agatttggag ctgcaggaaa agaattacag ttgagtacct ccagcacatt 2940
gtggaacaga aaaatggcaa agaaagagtg cccatcctct ggcatttcct gcagaaggaa 3000
gcagagctga ggctggtaaa gttcctgcct gagattttgg ccttgcaaag ggatctagtg 3060
aagcagttcc agaacgtcca gcaagttgaa tacagctcca tcagaggctt cctcagcaag 3120
cacagctcag atgggttgag gcagctgctt cacaacagga tcacagtctt tctgtccaca 3180
tggaacaaac tgaggagatc gcttgagacg aacggtgaga tcaacctacc caaagactac 3240
tgcagcactg acttggatct ggacactgag tttgagatcc tcttgccacg ccgacggggc 3300
ctgggcctct gtgctaccgc tctcgtcagc tacttgattc gcctacacaa tgaaattgtc 3360

tacgccgtgg aaaaactctc caaggaaaac aacagctatt ccgtggatgc cgccgaggtc 3420
actgaactgc atgtcatcag ttatgaagtg gagcgggacc tgactccact gattctctcc 3480
aactgccagt accaggtgga ggagggcaga gagaccgtgc aggagttcga tctggagaag 3540
attcagcggc agatcgtcag ccgcttcctc cagggcaagc cccggctgag cctcaaggga 3600
atacccactc tggtgtacag acacgactgg aactatgaac atctctttat ggacatcaag 3660
aacaaaatgg cacaggactc cctccccagc tcggtcatta gtgccatcag tggacagctg 3720
cagtcctaca gcgatgcctg tgaagtgctg tctgtcgtag aagtcactct ggggtttctg 3780
agcacagctg gtggggatcc aaacatgcag ctgaatgtgt atactcaaga catcctgcaa 3840
atgggtgatc agacgattca cgtgttaaag gccttaaaca gatgccagtt aaaacacacc 3900
attgccctct ggcagttcct gtctgctcat aagtctgaac agctgctgcg gctgcacaaa 3960
gagccatttg ggaaatcag ttcaaggtac aaagcggatc tgagcccgga aaatgctaag 4020
ctcctcagca cattcctaaa tcagactggc ctagacgcct tcctgctaga gctgcacgaa 4080
atgataatct tgaaactaaa gaaccccaa acccaaaccg aggagcgctt ccgccctcag 4140
tggagcctga gagacactct cgtaagttac atgcaaacta aagaaagtga aattcttcct 4200
gaaatggcat ctcagttccc agaagagata ctgctcgcca gctgtgtctc agtgtggaaa 4260
acagctgctg tgctgaaatg gaatcgagaa atgagatag 4299

<210> 3
<211> 1432
<212> PRT
<213> Homo sapiens

<400> 3
Met Arg Val Ser Thr Glu Glu Glu Leu Lys Phe Leu Gln Met Ala Leu
1 5 10 15

Trp Ser Cys Thr Arg Lys Leu Lys Ala Ala Ser Glu Ala Pro Glu Glu
20 25 30

Glu Val Ser Leu Pro Trp Val His Leu Ala Tyr Gln Arg Phe Arg Ser
35 40 45

Arg Leu Gln Asn Phe Ser Arg Ile Leu Thr Ile Tyr Pro Gln Val Leu
50 55 60

His Ser Leu Met Glu Ala Arg Trp Asn His Glu Leu Ala Gly Cys Glu
65 70 75 80

Met Thr Leu Asp Ala Phe Ala Ala Met Ala Cys Thr Glu Met Leu Thr
85 90 95

Arg Asn Thr Leu Lys Pro Ser Pro Gln Ala Trp Leu Gln Leu Val Lys
100 105 110

Asn Leu Ser Met Pro Leu Glu Leu Ile Cys Ser Asp Glu His Met Gln
115 120 125

Gly Ser Gly Ser Leu Ala Gln Ala Val Ile Arg Glu Val Arg Ala Gln
130 135 140

Trp Ser Arg Ile Phe Ser Thr Ala Leu Phe Val Glu His Val Leu Leu
145 150 155 160

Gly Thr Glu Ser Arg Val Pro Glu Leu Gln Gly Leu Val Thr Glu His
165 170 175

Val Phe Leu Leu Asp Lys Cys Leu Arg Glu Asn Ser Asp Val Lys Thr
180 185 190

His Gly Pro Phe Glu Ala Val Met Arg Thr Leu Cys Glu Cys Lys Glu
195 200 205

Thr Ala Ser Lys Thr Leu Ser Arg Phe Gly Ile Gln Pro Cys Ser Ile
210 215 220

Cys Leu Gly Asp Ala Lys Asp Pro Val Cys Leu Pro Cys Asp His Val
225 230 235 240

His Cys Leu Arg Cys Leu Arg Ala Trp Phe Ala Ser Glu Gln Met Ile
245 250 255

Cys Pro Tyr Cys Leu Thr Ala Leu Pro Asp Glu Phe Ser Pro Ala Val
260 265 270

Ser Gln Ala His Arg Glu Ala Ile Glu Lys His Ala Arg Phe Arg Gln
275 280 285

Met Cys Asn Ser Phe Phe Val Asp Leu Val Ser Thr Ile Cys Phe Lys
290 295 300

Asp Asn Ala Pro Pro Glu Lys Glu Val Ile Glu Ser Leu Leu Ser Leu
305 310 315 320

Leu Phe Val Gln Lys Gly Arg Leu Arg Asp Ala Ala Gln Arg His Cys
325 330 335

Glu His Thr Lys Ser Leu Ser Pro Phe Asn Asp Val Val Asp Lys Thr
340 345 350

Pro Val Ile Arg Ser Val Ile Leu Lys Leu Leu Leu Lys Tyr Ser Phe
355 360 365

His Asp Val Lys Asp Tyr Ile Gln Glu Tyr Leu Thr Leu Leu Lys Lys
370 375 380

Lys Ala Phe Ile Thr Glu Asp Lys Thr Glu Leu Tyr Met Leu Phe Ile
385 390 395 400

Asn Cys Leu Glu Asp Ser Ile Leu Glu Lys Thr Ser Ala Tyr Ser Arg
405 410 415

Asn Asp Glu Leu Asn His Leu Glu Glu Glu Gly Arg Phe Leu Lys Ala
420 425 430

Tyr Ser Pro Ala Ser Arg Gly Arg Glu Pro Ala Asn Glu Ala Ser Val
435 440 445

Glu Tyr Leu Gln Glu Val Ala Arg Ile Arg Leu Cys Leu Asp Arg Ala
450 455 460

Ala Asp Phe Leu Ser Glu Pro Glu Gly Gly Pro Glu Met Ala Lys Glu
465 470 475 480

Lys Gln Cys Tyr Leu Gln Gln Val Lys Gln Phe Cys Ile Arg Val Glu
485 490 495

Asn Asp Trp His Arg Val Tyr Leu Val Arg Lys Leu Ser Ser Gln Arg
500 505 510

Gly Met Glu Phe Val Gln Gly Leu Ser Lys Pro Gly Arg Pro His Gln
515 520 525

Trp Val Phe Pro Lys Asp Val Val Lys Gln Gln Gly Leu Arg Gln Asp
530 535 540

His Pro Gly Gln Met Asp Arg Tyr Leu Val Tyr Gly Asp Glu Tyr Lys
545 550 555 560

Ala Leu Arg Asp Ala Val Ala Lys Ala Val Leu Glu Cys Lys Pro Leu
565 570 575

Gly Ile Lys Thr Ala Leu Lys Ala Cys Lys Thr Pro Gln Ser Gln Gln
580 585 590

Ser Ala Tyr Phe Leu Leu Thr Leu Phe Arg Glu Val Ala Ile Leu Tyr
595 600 605

Arg Ser His Asn Ala Ser Leu His Pro Thr Pro Glu Gln Cys Glu Ala
610 615 620

Val Ser Lys Phe Ile Gly Glu Cys Lys Ile Leu Ser Pro Pro Asp Ile
625 630 635 640

Ser Arg Phe Ala Thr Ser Leu Val Asp Asn Ser Val Pro Leu Leu Arg
645 650 655

Ala Gly Pro Ser Asp Ser Asn Leu Asp Gly Thr Val Thr Glu Met Ala
660 665 670

Ile His Ala Ala Ala Val Leu Leu Cys Gly Gln Asn Glu Leu Leu Glu
675 680 685

Pro Leu Lys Asn Leu Ala Phe Ser Pro Ala Thr Met Ala His Ala Phe
690 695 700

Leu Pro Thr Met Pro Glu Asp Leu Leu Ala Gln Ala Arg Arg Trp Lys
705 710 715 720

Gly Leu Glu Arg Val His Trp Tyr Thr Cys Pro Asn Gly His Pro Cys
725 730 735

Ser Val Gly Glu Cys Gly Arg Pro Met Glu Gln Ser Ile Cys Ile Asp
740 745 750

Cys His Ala Pro Ile Gly Gly Ile Asp His Lys Pro Arg Asp Gly Phe
755 760 765

His Leu Val Lys Asp Lys Ala Asp Arg Thr Gln Thr Gly His Val Leu
770 775 780

Gly Asn Pro Gln Arg Arg Asp Val Val Thr Cys Asp Arg Gly Leu Pro
785 790 795 800

Pro Val Val Phe Leu Leu Ile Arg Leu Leu Thr His Leu Ala Leu Leu
805 810 815

Leu Gly Ala Ser Gln Ser Ser Gln Ala Leu Ile Asn Ile Ile Lys Pro
820 825 830

Pro Val Arg Asp Pro Lys Gly Phe Leu Gln Gln His Ile Leu Lys Asp
835 840 845

Leu Glu Gln Leu Ala Lys Met Leu Gly His Ser Ala Asp Glu Thr Ile
850 855 860

Gly Val Val His Leu Val Leu Arg Arg Leu Leu Gln Glu Gln His Gln
865 870 875 880

Leu Ser Ser Arg Arg Leu Leu Asn Phe Asp Thr Glu Leu Ser Thr Lys
885 890 895

Glu Met Arg Asn Asn Trp Glu Lys Glu Ile Ala Ala Val Ile Ser Pro
900 905 910

Glu Leu Glu His Leu Asp Lys Thr Leu Pro Thr Met Asn Asn Leu Ile
915 920 925

Ser Gln Asp Lys Arg Ile Ser Ser Asn Pro Val Ala Lys Ile Ile Tyr
930 935 940

Gly Asp Pro Val Thr Phe Leu Pro His Leu Pro Arg Lys Ser Val Val
945 950 955 960

His Cys Ser Lys Ile Trp Ser Cys Arg Lys Arg Ile Thr Val Glu Tyr
965 970 975

Leu Gln His Ile Val Glu Gln Lys Asn Gly Lys Glu Arg Val Pro Ile
980 985 990

Leu Trp His Phe Leu Gln Lys Glu Ala Glu Leu Arg Leu Val Lys Phe
995 1000 1005

Leu Pro Glu Ile Leu Ala Leu Gln Arg Asp Leu Val Lys Gln Phe Gln
1010 1015 1020

Asn Val Gln Gln Val Glu Tyr Ser Ser Ile Arg Gly Phe Leu Ser Lys
1025 1030 1035 1040

His Ser Ser Asp Gly Leu Arg Gln Leu Leu His Asn Arg Ile Thr Val
1045 1050 1055

Phe Leu Ser Thr Trp Asn Lys Leu Arg Arg Ser Leu Glu Thr Asn Gly
1060 1065 1070

Glu Ile Asn Leu Pro Lys Asp Tyr Cys Ser Thr Asp Leu Asp Leu Asp
1075 1080 1085

Thr Glu Phe Glu Ile Leu Leu Pro Arg Arg Arg Gly Leu Gly Leu Cys
1090 1095 1100

Ala Thr Ala Leu Val Ser Tyr Leu Ile Arg Leu His Asn Glu Ile Val
1105 1110 1115 1120

Tyr Ala Val Glu Lys Leu Ser Lys Glu Asn Asn Ser Tyr Ser Val Asp
1125 1130 1135

Ala Ala Glu Val Thr Glu Leu His Val Ile Ser Tyr Glu Val Glu Arg
1140 1145 1150

Asp Leu Thr Pro Leu Ile Leu Ser Asn Cys Gln Tyr Gln Val Glu Glu
1155 1160 1165

Gly Arg Glu Thr Val Gln Glu Phe Asp Leu Glu Lys Ile Gln Arg Gln
1170 1175 1180

Ile Val Ser Arg Phe Leu Gln Gly Lys Pro Arg Leu Ser Leu Lys Gly
1185 1190 1195 1200

Ile Pro Thr Leu Val Tyr Arg His Asp Trp Asn Tyr Glu His Leu Phe
1205 1210 1215

Met Asp Ile Lys Asn Lys Met Ala Gln Asp Ser Leu Pro Ser Ser Val
1220 1225 1230

Ile Ser Ala Ile Ser Gly Gln Leu Gln Ser Tyr Ser Asp Ala Cys Glu
1235 1240 1245

Val Leu Ser Val Val Glu Val Thr Leu Gly Phe Leu Ser Thr Ala Gly
1250 1255 1260

Gly Asp Pro Asn Met Gln Leu Asn Val Tyr Thr Gln Asp Ile Leu Gln
1265 1270 1275 1280

Met Gly Asp Gln Thr Ile His Val Leu Lys Ala Leu Asn Arg Cys Gln
1285 1290 1295

Leu Lys His Thr Ile Ala Leu Trp Gln Phe Leu Ser Ala His Lys Ser
1300 1305 1310

Glu Gln Leu Leu Arg Leu His Lys Glu Pro Phe Gly Glu Ile Ser Ser
1315 1320 1325

Arg Tyr Lys Ala Asp Leu Ser Pro Glu Asn Ala Lys Leu Leu Ser Thr
1330 1335 1340

Phe Leu Asn Gln Thr Gly Leu Asp Ala Phe Leu Leu Glu Leu His Glu
1345 1350 1355 1360

Met Ile Ile Leu Lys Leu Lys Asn Pro Gln Thr Gln Thr Glu Glu Arg
1365 1370 1375

Phe Arg Pro Gln Trp Ser Leu Arg Asp Thr Leu Val Ser Tyr Met Gln
1380 1385 1390

Thr Lys Glu Ser Glu Ile Leu Pro Glu Met Ala Ser Gln Phe Pro Glu
1395 1400 1405

Glu Ile Leu Leu Ala Ser Cys Val Ser Val Trp Lys Thr Ala Ala Val
1410 1415 1420

Leu Lys Trp Asn Arg Glu Met Arg
1425 1430

<210> 4
<211> 4616
<212> DNA
<213> Homo sapiens

<400> 4

ggatttcatt ctcttgacca tgcgtgtgtc aacggaggag gaattaaagt ttctgcagat 60
ggctctgtgg tcctgcacta ggaaactgaa agcggcgtca gaagcgcccg aggaagaggt 120
ttccttaccg tgggtgcacc ttgcctacca gcgtttcaga agccgtctgc agaacttttc 180
cagaatcctg accatctacc ctcaggttct ccacagcctg atggaagccc gttggaacca 240
tgagctggct ggatgtgaga tgaccctgga cgcatttgcc gcaatggcct gcacggagat 300
gctgacaaga aacaccctga agcccagtcc ccaggcgtgg ctacagttgg tgaagaatct 360
ttccatgccg ctggagctca tctgctccga tgagcacatg caaggcagcg ggagcctggc 420
ccaggctgtc atcagggaag tcagagccca gtggagtcgg attttctcca ccgcactctt 480
cgtggagcac gtgctcctag gaaccgagag ccgcgtcccc gagttacagg ggctggtgac 540
cgagcacgtc ttcttactag acaagtgtct tcgagagaac tctgacgtga agacgcacgg 600
gccttttgag gccgtgatgc gcactctctg tgaatgcaag gagacagcca gcaagaccct 660
cagcaggttt gggattcagc cgtgctccat ctgcctggga gatgcaaagg accccgtctg 720
tctgccctgc gaccacgtgc actgcctgcg ctgcctcagg gcctggtttg cctcagagca 780
gatgatatgc ccctactgtt taactgcctt gccagacgaa ttctctccag ctgtttccca 840
agcgcacagg gaagccattg aaaagcatgc ccgcttccgg cagatgtgca acagtttctt 900
cgtagacctg gtgtccacca tttgcttcaa ggacaacgct ccgcctgaga aggaagtgat 960
tgagagcctg ctctctctcc tcttcgtcca aaaggggcgc ttaagagatg ctgcccagag 1020
acactgtgaa cacacaaaat ctctctctcc attcaatgat gttgtggata agactcctgt 1080
catccgctca gtgatactga aactgctttt gaagtacagc tttcatgatg taaaagatta 1140
tattcaggaa tatttgaccc tgttaaaaaa gaaagcattc ataactgaag ataaaactga 1200
actgtacatg ctcttcatca actgcctgga ggattcaata cttgagaaga ccagtgctta 1260
ctccagaaat gatgaactga accacctaga agaggaaggt cgtttcctta aggcatattc 1320
tccagcaagc cggggccgag agcctgccaa cgaggcctcg gttgaatacc tgcaagaggt 1380
ggcccggatc cgcctctgcc tcgacagagc tgcagatttc ctctcggagc ctgagggagg 1440
cccagagatg gccaaggaga agcagtgcta cctgcagcaa gtcaagcagt tctgtatccg 1500
ggtggagaac gactggcacc gggtgtacct ggtgcggaag ctcagcagcc agcgggggat 1560
ggagttcgtg cagggcctct ccaagcccgg ccgcccgcac cagtgggtgt ttcccaagga 1620
cgttgtcaag cagcaggggc tgcggcagga ccacccaggc cagatggata ggtacctggt 1680
gtacggcgat gaatacaagg ctctccgtga tgctgtggcc aaagctgtcc tcgagtgcaa 1740

gccactgggc attaagactg ctctgaaggc ctgcaagacc ccccaaagcc agcagtcagc 1800
ctacttcctg ttaacactgt ttagagaggt ggctattttg tacagatccc acaatgcaag 1860
cctccacccc acgccagagc aatgtgaagc tgtgagcaaa ttcattggcg aatgcaagat 1920
cctttcacct cctgatatca gccgttttgc aacatcgctc gtggacaatt ctgtgccatt 1980
gttgagggcg gggcctagtg acagcaacct tgatggaacg gtgacagaaa tggccattca 2040
tgctgcagcc gtccttctgt gtggacagaa tgaactcttg gagcccctaa agaatctggc 2100
cttctcccca gccaccatgg cgcatgcttt tcttccaacc atgcctgaag acttgctggc 2160
tcaagctcgg aggtggaagg gtctggagcg agtccactgg tacacttgtc ccaacggcca 2220
tccttgctcc gtgggagagt gtggcaggcc gatggaacag agcatctgca ttgactgcca 2280
tgcgccgatt ggaggcattg accacaaacc tcgggacggc tttcatctgg tcaaagacaa 2340
ggcagacaga acgcagaccg gccacgtgct gggcaacccg cagcggagag acgtggtgac 2400
atgtgaccga gggctgcccc cagtggtctt cctccttatc cggctactca ctcacttggc 2460
tctgcttctg ggagcgtccc agagttccca ggctctgata aacatcatta agcctccagt 2520
gagggatcca aaaggctttc tgcagcagca catcctgaag gacctggagc agttggccaa 2580
gatgctggga cacagtgccg acgagaccat cggcgtggtc cacctcgtcc tgcgcaggct 2640
tctccaagag cagcaccagc tctctagcag aaggctttta aattttgaca cagaattgtc 2700
aactaaagaa atgaggaaca actgggaaaa ggaaatcgca gctgtgattt ctcctgaact 2760
ggagcatcta gataaaaccc ttcccaccat gaataatctc atcagccaag ataagcgtat 2820
cagctctaac cctgtggcca aaataatata tggtgaccca gtgaccttcc tgccccacct 2880
gccccggaaa agtgtggtcc attgctctaa gatttggagc tgcaggaaaa gaattacagt 2940
tgagtacctc cagcacattg tggaacagaa aaatggcaaa gaaagagtgc ccatcctctg 3000
gcatttcctg cagaaggaag cagagctgag gctggtaaag ttcctgcctg agattttggc 3060
cttgcaaagg gatctagtga agcagttcca gaacgtccag caagttgaat acagctccat 3120
cagaggcttc ctcagcaagc acagctcaga tgggttgagg cagctgcttc acaacaggat 3180
cacagtcttt ctgtccacat ggaacaaact gaggagatcg cttgagacga acggtgagat 3240
caacctaccc aaagactact gcagcactga cttggatctg gacactgagt ttgagatcct 3300
cttgccacgc cgacggggcc tgggcctctg tgctaccgct ctcgtcagct acttgattcg 3360
cctacacaat gaaattgtct acgccgtgga aaaactctcc aaggaaaaca acagctattc 3420
cgtggatgcc gccgaggtca ctgaactgca tgtcatcagt tatgaagtgg agcgggacct 3480
gactccactg attctctcca actgccagta ccaggtggag gagggcagag agaccgtgca 3540

ggagttcgat ctggagaaga ttcagcggca gatcgtcagc cgcttcctcc agggcaagcc 3600
ccggctgagc ctcaagggaa tacccactct ggtgtacaga cacgactgga actatgaaca 3660
tctctttatg gacatcaaga acaaaatggc acaggactcc ctccccagct cggtcattag 3720
tgccatcagt ggacagctgc agtcctacag cgatgcctgt gaagtgctgt ctgtcgtaga 3780
agtcactctg gggtttctga gcacagctgg tggggatcca aacatgcagc tgaatgtgta 3840
tactcaagac atcctgcaaa tgggtgatca gacgattcac gtgttaaagg ccttaaacag 3900
atgccagtta aaacacacca ttgccctctg gcagttcctg tctgctcata agtctgaaca 3960
gctgctgcgg ctgcacaaag agccatttgg ggaaatcagt tcaaggtaca aagcggatct 4020
gagcccggaa aatgctaagc tcctcagcac attcctaaat cagactggcc tagacgcctt 4080
cctgctagag ctgcacgaaa tgataatctt gaaactaaag aacccccaaa cccaaaccga 4140
ggagcgcttc cgccctcagt ggagcctgag agacactctc gtaagttaca tgcaaactaa 4200
agaaagtgaa attcttcctg aaatggcatc tcagttccca gaagagatac tgctcgccag 4260
ctgtgtctca gtgtggaaaa cagctgctgt gctgaaatgg aatcgagaaa tgagatagaa 4320
ttatttcctc agctatcttt ggatgacttt ggagagaaga ctcctctctc ctcgtctgcg 4380
gcgtggactt gatcatggac tggtgccttt gcattcagaa ggagagctgt cagcgtagca 4440
ccgaattcaa gaccaaggcg tgctacctga gctgacagct ttttgaaagc cgagctgttt 4500
ctgaaccatg tacatacatg ttctgaaact ttctcatcat tttatgagta ctgttcattg 4560
agagatgaca atgaagatta gatgaaattg gaaataaacc aacattgttt acattc 4616